# EUROPEAN PATENT SPECIFICATION

(11) **EP 0 655 088 B1**
(45) Date of publication and mention of the grant of the patent: **24.07.2002**
(21) Application number: 92922127.3
(22) Date of filing: 14.10.1992
(51) Int. Cl.: C12P 19/34, C07K 2/00, C07H 21/00, C07D 473/30, C07D 473/34, C07H 19/10, C07H 19/20

(54) **OLIGONUCLEOTIDES HAVING CHIRAL PHOSPHORUS LINKAGES**
ÜBER CHIRALE PHOSPHORATOME GEBUNDENE OLIGONUKLEOTIDE
OLIGONUCLEOTIDES PRESENTANT DES LIAISONS PHOSPHOREES CHIRALES

(30) Priority: 15.10.1991 US 777670; 16.10.1991 US 777007
(43) Date of publication of application: 31.05.1995
(73) Proprietor: ISIS PHARMACEUTICALS, INC., Carlsbad, CA 92008 (US)
(72) Inventor: HOKE, Glenn, D., Gaithersburg, MD 20878 (US); COOK, Phillip, Dan, Carlsbad, CA 92009 (US)
(74) Representative: Hallybone, Huw George
(86) International application number: PCT/US92/08797
(87) International publication number: WO 93/08296

(56) References cited:
- EP-A- 0 506 242
- NUCLEOSIDES AND NUCLEOTIDES, vol.6, no.1-2, 1987 pages 451 - 456 F.SEELA ET AL. 'Phosphoramidites of (oxygen-18) Chiral (Rp)- and (Sp)-configurated Dimer-blocks and their use in Automated Oligonucleotide Synthesis'
- BIOCHEMISTRY., vol.10, 1971, EASTON, PA US pages 186 - 187 H.FOLLMANN ET AL. 'Interaction of Ribonucleotide Reductase with Ribonucleotide Analogs'
- NUCLEIC ACIDS RESEARCH., vol.19, no.21, 11 November 1991, ARLINGTON, VIRGINIA US pages 5883 - 5888 W.J.STEC ET AL. 'Novel Route to Oligo(deoxyribonucleoside phosphorothioates). Stereocontrolled Synthesis of P-chiral Oligo(deoxyribonucleoside phosphorothioates)'
- J. COHEN, "Oligonucleotides", published 1989 by CRC Press, Inc. (Florida), see pages 7-116, 137-210.
- The Journal of Biological Chemistry, Volume 257, No. 13, issued 10 July 1982, A. GUPTA et al., "Template-Primer Dependent Turnover of (Sp) - dATPaS by T4 DNA Polymerase", see pages 7689-7692.
- The Journal of Biological Chemistry, Vol. 13, issued 10 July 1982, P.J. ROMANIUK et al., "A Study of the Mechanism of T4 DNA Polymerase with Diastereomeric Phosphorothioate Analogues of Deoxyadenosine Triphosphate", see pages 7684-7688.
- Tetrahedron Letters 30, 29, 1989, 3821-3824

### SEQUENCE LISTING

(1) GENERAL INFORMATION:
   (i) APPLICANT: Cook, Philip Dan Hoke, Glen Dale, Jr.
   (ii) TITLE OF INVENTION: Oligonucleotides Having Chiral Phosphorus
   (iii) NUMBER OF SEQUENCES: 10
   (iv) CORRESPONDENCE ADDRESS:
      (A) ADDRESSEE: Woodcock Washburn Kurtz Mackiewicz and Norris
      (B) STREET: One Liberty Place - 46th Floor
      (C) CITY: Philadelphia
      (D) STATE: PA
      (E) COUNTRY: U.S.A.
      (F) ZIP: 19103
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Gaumond, Rebecca Ralph
      (B) REGISTRATION NUMBER: 35,152
      (C) REFERENCE/DOCKET NUMBER: ISIS-
   (ix) TELECOMMUNICATION INFORMATION:
      (A) TELEPHONE: 215-368-3100
      (B) TELEFAX: 215-568-3439
(2) INFORMATION FOR SEQ ID NO:1:
   (i) SEQUENCE CHARACTERISTICS:
      (A) LENGTH: 18 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:1:
   GTCCGCGTCC ATGTCGGC 18
(2) INFORMATION FOR SEQ ID NO:2:
   (i) SEQUENCE CHARACTERISTICS:
      (A) LENGTH: 18 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:2:
   GCTCCCAGGC TCAGATCT 18
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:3:
   TGTTGTCGAT AATATTACCA 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:4:
   TTGCTTCCAT CTTCCTCGTC 20
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:5:
   TCCGTCATCG CTCCTCAGGG 20
(2) INFORMATION FOR SEQ ID NO:6:
   (i) SEQUENCE CHARACTERISTICS:
      (A) LENGTH: 18 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:6:
   TGGGAGCCAT AGCGAGGC 18
(2) INFORMATION FOR SEQ ID NO:7:
   (i) SEQUENCE CHARACTERISTICS:
      (A) LENGTH: 55 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:7:
   GTACTTGCAT AGTCGATCGG AAAATAGGGT TCTCATCTCC CGGGATTTGG TTGAG
(2) INFORMATION FOR SEQ ID NO:8:
   (i) SEQUENCE CHARACTERISTICS:
      (A) LENGTH: 41 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:8:
   CTCAACCAAA TCCCGGGAGA TGAGAACCCT ATTTTCCGAT C 41
(2) INFORMATION FOR SEQ ID NO:9:
   (i) SEQUENCE CHARACTERISTICS:
      (A) LENGTH: 15 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:9:
   CGACTATGCA AGTAC 15
(2) INFORMATION FOR SEQ ID NO:10:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   GACTATGCAA GTAC 14

## Description

### CROSS REFERENCE TO RELATED APPLICATIONS:

Portions of this application may have been supported by National Institute of Health Grant No. GM45061.

### FIELD OF THE INVENTION:

This invention is directed to sequence-specific oligonucleotides having chiral phosphorus,linkages and to a novel enzymatic and chemical synthesis of these and other oligonucleotides. The invention includes chiral alkylphosphonate, chiral phosphotriester, chiral phosphorothioates, and chiral phosphoramidate-linked oligonucleotides. The invention further includes chiral phosphorothioate, chiral alkylphosphonate, chiral phosphotriester, and chiral phosphoramidate-linked oligonucleotides that contain at least one modified nucleoside unit. The novel chemical synthesis provides such chiral phosphorothioate, chiral alkylphosphonate, chiral phosphotriester, and chiral phosphoramidate oligonucleotides as well as "natural" or "wild type" phosphodiester oligonucleotides.

### BACKGROUND OF THE INVENTION:

Messenger RNA (mRNA) directs protein synthesis. As a therapeutic strategy, antisense therapy strives to disrupt the synthesis of target proteins by using a sequence-specific oligonucleotide to form a stable heteroduplex with its corresponding mRNA. Such antisense oligonucleotides generally have been natural phosphodiester oligonucleotides.

As contrasted to natural phosphodiester oligonucleotides, the use of phosphorothioate, methylphosphonate, phosphotriester or phosphoramidate oligonucleotides in antisense therapy provides certain distinguishing features. Each of the phosphorothioate, methylphosphonate, phosphotriester or phosphoramidate phosphorus linkages can exist as diastereomers. Certain of these phosphorothioate, methylphosphonate, phosphotriester or phosphoramidate oligonucleotides have a greater resistance to nucleases. Some have solubilities similar to the solubility of natural phosphodiester oligonucleotides. Other have solubilities different from that of the natural phosphodiester oligonucleotides. Some are generally more chemically or thermodynamically stable than the natural phosphodiester oligonucleotides. At least the phosphorothioates have oligonucleotide-RNA heteroduplexes that can serve as substrates for endogenous RNase H.

The phosphorothioate oligonucleotides, like the natural phosphodiester oligonucleotides, are soluble in aqueous media. In contrast, methylphosphonate, phosphotriester, and phosphoramidate oligonucleotides, which lack a charge on the phosphorus group, can penetrate cell membranes to a greater extent and, thus, facilitate cellular uptake. The internucleotide linkage in methylphosphonate oligonucleotides is more base-labile than that of the natural phosphodiester internucleotide linkage, while the internucleotide linkage of the phosphorothioate oligonucleotides is more stable than the natural phosphodiester oligonucleotide linkage.

The resistance of phosphorothioate oligonucleotides to nucleases has been demonstrated by their long half-life in the presence of various nucleases relative to natural phosphodiester oligonucleotides. This resistance to nucleolytic degradation *in vitro* also applies to *in vivo* degradation by endogenous nucleases. This *in vivo* stability has been attributed to the inability of 3'-5' plasma exonucleases to degrade such oligonucleotides. Phosphotriester and methylphosphonate oligonucleotides also are resistant to nuclease degradation, while phosphoramidate oligonucleotides show some sequence dependency.

Since they exist as diastereomers, phosphorothioate, methylphosphonate, phosphotriester or phosphoramidate oligonucleotides synthesized using known, automated techniques result in racemic mixtures of Rp and Sp diastereomers at the individual phosphorothioate, methylphosphonate, phosphotriester or phosphoramidate linkages. Thus, a 15-mer oligonucleotide containing 14 asymmetric linkages has 2¹⁴, i.e. 16,384, possible stereoisomers. Accordingly, it is possible that only a small percentage of the oligonucleotides in a racemic mixture will hybridize to a target mRNA or DNA with sufficient affinity to prove useful in antisense or probe technology.

Miller, P.S., McParland, K.B., Jayaraman, K., and Ts'o, P.O.P (1981), *Biochemistry,* 20:1874, found that small di-, tri- and tetramethylphosphonate and phosphotriester oligonucleotides hybridize to unmodified strands with greater affinity than natural phosphodiester oligonucleotides. Similar increased hybridization was noted for small phosphotriester and phosphoramidate oligonucleotides; Koole, L.H., van Genderen, M.H.P., Reiners, R.G., and Buck, H.M. (1987), *Proc. K. Ned*. *Adad*. *Wet.,* 90:41; Letsinger, R.L., Bach, S.A., and Eadie, J.S. (1986), *Nucleic Acids Res.,* 14:3487; and Jager, A., Levy, M.J., and Hecht, S.M. (1988), *Biochemistry,* 27:7237. The effects of the racemic diastereomers on hybridization becomes even more complex as chain length increases.

Bryant, F.R. and Benkovic, S.J. (1979), *Biochemistry*, 18:2825 studied the effects of diesterase on the diastereomers of ATP. Published patent application WO 89/03683 (PCT/US88/03634) discloses dimers and trimers of 2'-5'- linked diastereomeric adenosine units. Niewiarowski, W., Lesnikowski, Z.J., Wilk, A., Guga, P., Okruszek, A., Uznanski, B., and Stec, W. (1987), *Acta Biochimica Polonia,* 34:217, synthesized diastereomeric dimers of thymidine, as did Fujii, M., Ozaki, K., Sekine, M., and Hata, T. (1987), *Tetrahedron,* 43:3395.

Stec, W.J., Zon, G., and Uznanski, B. (1985), *J*. *Chromatography,* 326:263, have reported the synthesis of certain racemic mixtures of phosphorothioate or methyphosphonate oligonucleotides. However, they were only able to resolve the diastereomers of certain small oligomers having one or two diastereomeric phosphorus linkages.

In a preliminary report, J.W. Stec, *Oligonucleotides as antisense inhibitors of gene expression: Therapeutic implications,* meeting abstracts, June 18-21, 1989, noted that a non-sequence-specific thymidine homopolymer octomer -- i.e. a (dT)₈-mer, having "all-except-one" Rp configuration methylphosphonate linkages -- formed a thermodynamically more stable hybrid with a 15-mer deoxyadenosine homopolymer -- i.e. a d(A)₁₅-mer -- than did a similar thymidine homopolymer having "all-except-one" Sp configuration methylphosphonate linkages. The hybrid between the "all-except-one" Rp (dT)₈-mer and the d(A)₁₅-mer had a Tₘ of 38°C while the Tₘ of the "all-except-one" Sp (dT)₈-mer and the d(A)₁₅-mer was < 0°C. The hybrid between a (dT)₈-mer having natural phosphodiester linkages, i.e. octathymidylic acid, and the d(A)₁₅-mer was reported to have a Tₘ of 14°C. The "all-except-one" thymidine homopolymer octamers were formed from two thymidine methylphosphonate tetrameric diastereomers linked by a natural phosphodiester linkage.

To date, it has not been possible to chemically synthesize an oligonucleotide having more than two adjacent, chirally pure phosphorous linkages. Indeed, even in homopolymers it has been possible to produce only three such adjacent chiral linkages. For an oligonucleotide to be useful as an antisense compound, many nucleotides must be present. While not wishing to be bound by any particular theory, it is presently believed that generally at least about 10 or more nucleotides are necessary for an oligonucleotide to be of optimal use as an antisense compound. Because it has not been possible to resolve more than two or three adjacent phosphorus linkages, the effects of induced chirality in the phosphorus linkages of chemically synthesized antisense oligonucleotides has not been well assessed heretofore.

Except as noted above, the sequence-specific phosphorothioate, methylphosphonate, phosphotriester or phosphoramidate oligonucleotides obtained utilizing known automated synthetic techniques have been racemic mixtures. Indeed, it was recently stated in a review article that: "It is not yet possible to synthesize by chemical means diastereomerically pure chains of the length necessary for antisense inhibition," see J. Goodchild (1990) *Bioconjugate Chemistry,* 1:165.

The use of enzymatic methods to synthesize oligonucleotides having chiral phosphorous linkages has also been investigated. Burgers, P.M.J. and Eckstein, F. (1979), *J*. *Biological Chemistry,* 254:6889; and Gupta, A., DeBrosse, C., and Benkovic, S.J. (1982) *J. Bio. Chem.,* 256:7689 enzymatically synthesized diastereomeric polydeoxyadenylic acid having phosphorothioate linkages. Brody, R.S. and Frey, P.S. (1981), *Biochemistry,* 20:1245; Eckstein, F. and Jovin, T.M. (1983), *Biochemistry,* 2:4546; Brody, R.S., Adler, S., Modrich, P., Stec, W.J., Leznikowski, Z.J., and Frey, P.A. (1982) *Biochemistry,* 21: 2570-2572; and Romaniuk, P.J. and Eckstein, F. (1982) *J. Biol*. *Chem.,* 257:7684-7688 all enzymatically synthesized poly TpA and poly ApT phosphorothioates while Burgers, P.M.J. and Eckstein, F. (1978) *Proc. Natl*. *Acad*. *Sci*. *USA*, 75: 4798-4800 enzymatically synthesized poly UpA phosphorothioates. Cruse, W.B.T., Salisbury, T., Brown, T., Cosstick, R. Eckstein, F., and Kennard, O. (1986), *J. Mol. Biol*., 192:891, linked three diastereomeric Rp GpC phosphorothioate dimers via natural phosphodiester bonds into a hexamer. Most recently Ueda, T., Tohda, H., Chikazuni, N., Eckstein, R., and Watanabe, K. (1991) *Nucleic Acids Research,* 19:547, enzymatically synthesized RNA's having from several hundred to ten thousand nucleotides intermittently incorporating Rp diastereomeric phosphorothioate linkages. European patent application EP-A-0506242 has a priority date of 6th March 1991, but was published on 30th September 1992, after the filing date of this European application. It discloses methods and compounds for solid phase synthesis of oligonucleotides and oligonucleotide analogues. The chiral oligonucleotides have nucleoside linkages which are either all Rp or all Sp.

J. Cohen, "Oligonucleotides", 1989, CRC Press, Inc. (Florida), pp 7-116 & 137-210 describes, generally, oligonucleotides containing chiral phosphate linkages.

Biochemistry, 1971, 10, 186-187 describes a series of ribonucleotide analogues. These compounds do not contain a leaving group at the 3' position.

While phosphorothioate, alkylphosphonate, phosphoamidate, and phosphotriester oligonucleotides have useful characteristics, little is known concerning the effects of differing chirality at the phosphorus linkages. It would therefore be of great advantage to provide oligonucleotides having phosphorous linkages of controlled stereochemistry.

### OBJECTS OF THE INVENTION:

Accordingly, it is one object of this invention to provide sequence-specific oligonucleotides having chirally pure phosphorothioate, alkylphosphonate, phosphotriester or phosphoramidate linkages.

It is a further object to provide phosphorothioate, alkylphosphonate, phosphoramidate, and phosphotriester oligonucleotides comprising substantially all Rp or all Sp linkages.

It is another object to provide phosphorothioate, alkylphosphonate, phosphoramidate, and phosphotriester oligonucleotides that have antisense hybridizability against DNA and RNA sequences.

It is still another object of this invention to provide phosphorothioate, alkylphosphonate, phosphoramidate, and phosphotriester oligonucleotides for use in antisense diagnostics and therapeutics.

A further object is to provide research and diagnostic methods and materials for assaying bodily states in animals, especially diseased states.

Another object is to provide therapeutic and research methods and materials for the treatment of diseases through modulation of the activity of DNA and RNA.

It is yet another object to provide new methods for synthesizing sequence-specific oligonucleotides having chirally pure phosphorothioate, methylphosphonate, phosphotriester or phosphoramidate linkages.

### SUMMARY OF THE INVENTION:

The present invention provides stereoselective methods for preparing sequence-specific oligonucleotides having chiral phosphorous linkages. In certain preferred embodiments, these methods comprise the steps of:
(a) selecting a first synthon having structure (1): 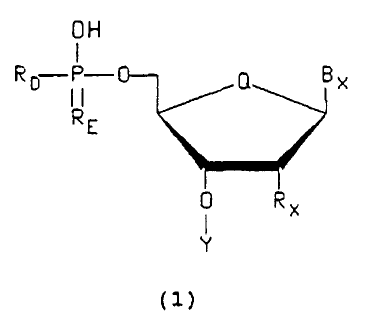 wherein:
   Q is O or CH₂;
   R_{D} is O^{⊖}, S^{⊖}, methyl, O-alkyl, S-alkyl, amino or substituted amino;
   R_{E} is O or S;
   R_{X} is H, OH, or a sugar derivatizing group;
   B_{X} is a naturally occurring or synthetic nucleoside base or blocked nucleoside base; and
   Y is a stable blocking group, a solid state support, a nucleotide on a solid state support, or an oligonucleotide on a solid state support;
(b) selecting a second synthon having structure (2): 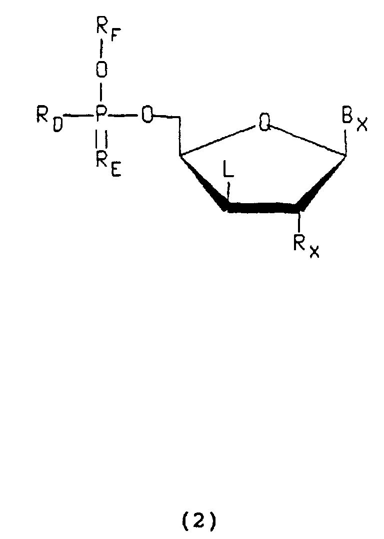 wherein:
   R_{F} is a labile blocking group; and
   L is a leaving group or together L and B_{X} are a 2-3' or 6-3' pyrimidine or 8-3' purine cyclonucleoside;
(c) adding the second synthon to the first synthon in the presence of a base to effect nucleophilic attack of the 5'-phosphate of the first synthon at the 3'-position of the second synthon to yield a new first synthon having structure (3): 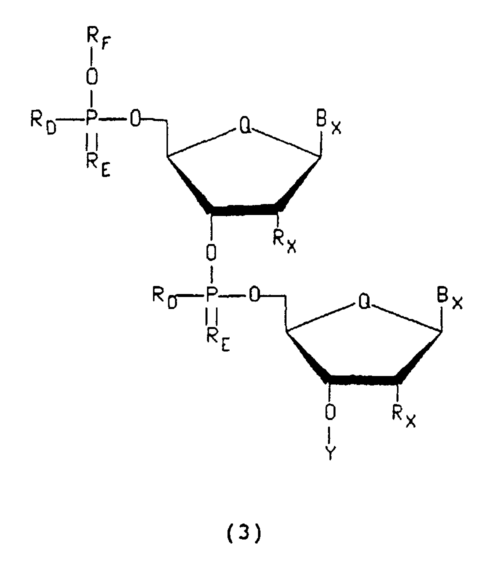 via a stereospecific inversion of configuration at the 3' position of the second synthon; and
(d) treating the new first synthon with a reagent to remove the labile blocking group R_{F}.

Additional nucleotides are added to the new first synthon by repeating steps (b), (c), and (d) for each additional nucleotide. Preferably, R_{F} is an acid-labile blocking group and said new first synthon in step (d) is treated with an acidic reagent to remove said acid-labile R_{F} blocking group.

The present invention also provides enzymatic methods for preparing oligonucleotides comprising nucleoside units joined together by either substantially all Sp or substantially all Rp phosphorus intersugar linkages comprising combining a sequence primer and a template and adding an excess of all four nucleoside triphosphates having a desired 5'phosphorus moiety; said triphosphate being all Rp or all Sp triphosphates. Alternatively, an excess of racemic mixture of all four triphosphates and a selected metal ion effective to promote preferential incorporation of either Rp or Sp triphosphates are added. Polymerase is added to extend the primer to form oligonucleotide complementary to said template and the resulting oligonucleotide are disassociated from the primer and from the template.

In another embodiment of the present invention are provided sequence specific oligonucleotides having at least 10 units linked together and complementary to at least a portion of a sequence of a targeted RNA or DNA, wherein at least three of said nucleoside units are linked together by either substantially all Rp or all Sp phosphorothioate, alkylphosphonate, phosphotriester, or phosphoramidate, with the proviso that if said oligonucleotide is linked together by an phosphorothioate linkages then said oligonucleotide has less than all of said nucleoside units linked together by either all Rp or all Sp linkages.

The present invention also provides oligonucleotides comprising at least 10 nucleoside units linked together via phosphate linkages, wherein:
at least one of the nucleoside units is a non-naturally occurring nucleoside unit having at the 2' position a substituent selected from the group consisting of OH, C₁₋₁₂ alkyl, C₁₋₁₂ substituted alkyl, F, Cl, Br, CN, CF₃, OCF₃, OCN, O-alkyl, substituted O-alkyl, S-alkyl, substituted S-alkyl, SOMe, SO₂Me, ONO₂, NO₂, N₃, NH₂, NH-alkyl, substituted NH-alkyl, OCH₂CH=CH₂, OCH=CH₂, OCH₂CCH, OCCH, aralkyl, heteroaralkyl, heterocycloalkyl, poly-alkylamino, substituted silyl, an RNA cleaving moiety, a group which improves the pharmacodynamic properties of an oligonucleotide, or a group which improves the pharmacokinetic properties of an oligonucleotide; and
at least two of the units are linked via chiral phosphate linkages.

In preferred embodiments of the invention, the oligonucleotides include non-naturally occurring nucleoside units incorporated into the oligonucleotide chain. Such nucleoside units preferably have structure (4) or structure (5): 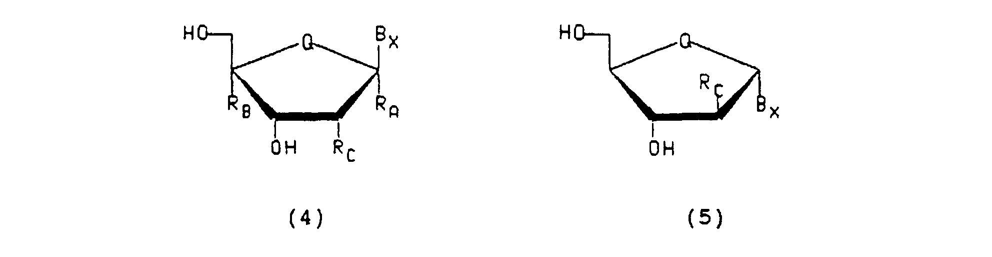 wherein Q is O or CHR_{G}, R_{A} and R_{B} are H, lower alkyl, substituted lower alkyl, an RNA cleaving moiety, a group which improves the pharmacodynamic properties of an oligonucleotide, or a group which improves the pharmacokinetic properties of an oligonucleotide; B_{X} is a naturally occuring or synthetic nucleoside base; R_{C} is H, OH, lower alkyl, lower alkenyl, lower alkynyl, substituted lower alkyl, substituted lower alkenyl, substituted lower alkynyl, F, Cl, Br, CN, CF₃, OCF₃, OCN, O-alkyl, O-alkenyl, 0-alkynyl, substituted 0-alkyl, substituted O-alkenyl, substituted O-alkynyl, S-alkyl, S-alkenyl, S-alkynyl, substituted S-alkyl, substituted S-alkenyl, substituted S-alkynyl, SOMe, SO₂Me, ONO₂, NO₂, N₃, NH₂, NH-alkyl, NH-alkenyl, NH-alkynyl, substituted NH-alkyl, substituted NH-alkenyl, substituted NH-alkynyl, OCH₂CH=CH₂, OCH=CH₂, OCH₂CCH, OCCH, aralkyl, aralkenyl, aralkynyl, heteroaralkyl, heteroaralkenyl, heteroaralkynyl, heterocycloalkyl, poly-alkylamino, substituted silyl, an RNA cleaving moiety, a group which improves the pharmacodynamic properties of an oligonucleotide, or a group which improves the pharmacokinetic properties of an oligonucleotide; and R_{G} is H, lower alkyl, substituted lower alkyl, an RNA cleaving moiety, a group which improves the pharmacodynamic properties of an oligonucleotide, or a group which improves the pharmacokinetic properties of an oligonucleotide.

In preferred embodiments, B_{X} is a pyrimidinyl-1 or purinyl-9 moiety such as, for example, adenine, guanine, hypoxanthine, uracil, thymine, cytosine, 2-aminoadenine or 5-methylcytosine.

In further preferred embodiments, the modified nucleosides include nucleosides having structures (6)-(11): 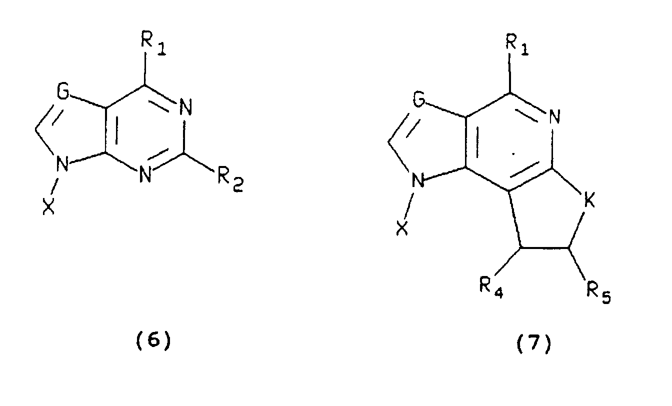 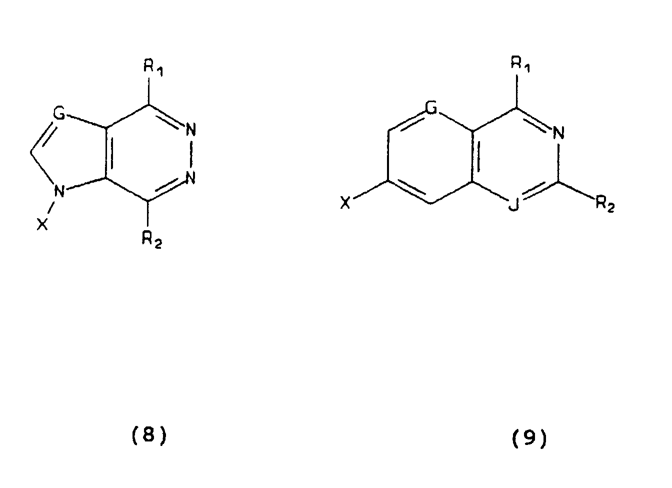 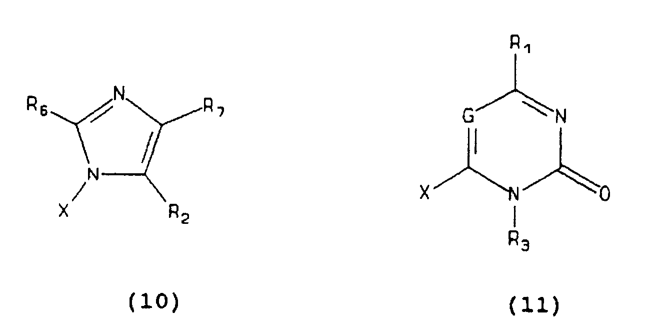 wherein:
G and K are, independently, C or N;
J is N or CR₂R₃;
R₁ is OH or NH₂;
R₂ and R₃ are H, NH, lower alkyl, substituted lower alkyl, lower alkenyl, aralkyl, alkylamino, aralkylamino, substituted alkylamino, heterocycloalkyl, heterocycloalkylamino, aminoalkylamino, hetrocycloalkylamino, polyalkylamino, an RNA cleaving moiety, a group which improves the pharmacokinetic properties of an oligonucleotide, or a group which improves the pharmacodynamic properties of an oligonucleotide;
R₄ and R₅ are, independently, H, OH, NH₂, lower alkyl, substituted lower alkyl, substituted amino, an RNA cleaving moiety, a group which improves the pharmacokinetic properties of an oligonucleotide, or a group which improves the pharmacodynamic properties of an oligonucleotide;
R₆ and R₇ are, independently, H, OH, NH₂, SH, halogen, CONH₂, C(NH)NH₂, C(O)O-alkyl, CSNH₂, CN, C(NH)NHOH, lower alkyl, substituted lower alkyl, substituted amino, an RNA cleaving moiety, a group which improves the pharmacokinetic properties of an oligonucleotide, or a group which improves the pharmacodynamic properties of an oligonucleotide; and
X is a sugar or a sugar substituted with at least one substituent comprising an RNA cleaving moiety, a group which improves the pharmacodynamic properties of an oligonucleotide, or a group which improves the pharmacokinetic properties of an oligonucleotide.

The present invention also provides compounds which are useful in forming the oligonucleotides of invention. Such compounds have structure (12): 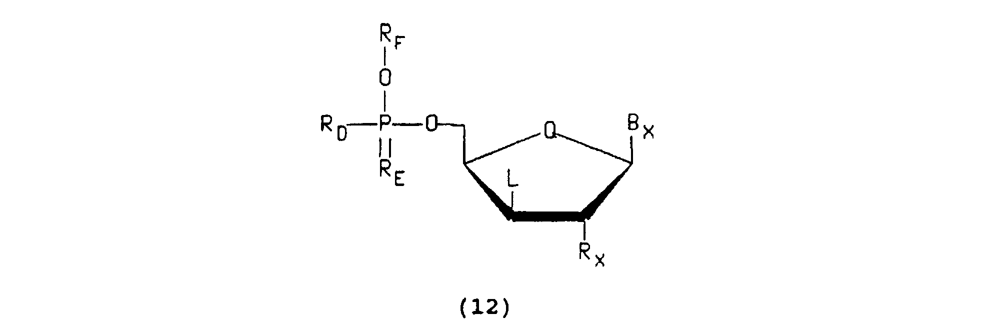 wherein Q is O or CH₂; R_{D}, R_{E}, R_{X}, L, and B_{X} are defined as above and R_{F} is H or a labile blocking group.

The oligonucleotides of the invention are useful to increase the thermodynamic stability of heteroduplexes with target RNA and DNA. Certain of the oligonucleotides of the invention are useful to elicit RNase H activity as a termination event. Certain other oligonucleotides are useful to increase nuclease resistance. The oligonucleotides of the invention are also useful to test for antisense activity using reporter genes in suitable assays and to test antisense activity against selected cellular target mRNA's in cultured cells. The production of protein may be modulated by contacting a cell with an oligonucleotide of the present invention wherein said oligonucleotide is complementary to at least a portion of a sequence of targeted RNA or DNA involved in the production of said protein.

### DETAILED DESCRIPTION OF THE INVENTION:

As will be recognized, adjacent nucleosides of a naturally occurring or wild type oligonucleotide are joined together by phosphodiester linkages, i.e. diesters of phosphoric acid. The natural phosphodiester linkages in oligonucleotides are at the same time both non-chiral and prochiral sites. Substitution of one of the oxygen atoms of the phosphate moiety of a nucleotide with another atom yields an asymmetric center on the phosphorus atom. Since a nucleotide unit already contains a first asymmetrical center within its sugar moiety, further asymmetry at the phosphorus atom of the nucleotide yields a diasymmetric nucleotide. Such a diasymmetric nucleotide is a chiral nucleotide having Sp and Rp diastereomers.

Substitution of one of the oxygen atoms of the phosphate moiety of a nucleotide with a sulfur atom yields Sp and Rp diastereomeric phosphorothioate analogs. Similarly, substitution of a phosphate oxygen atom by an alkyl moiety yields diastereomeric alkylphosphonate analogs. Substitution with an alkoxy group yields diastereomeric Sp and Rp phosphotriesters. Substitution with a thioalkoxy group yields a mixed triester -- a phosphodiesterthioester. Substitution with an amine or a substituted amine (including heterocyclic amines) yields diastereomeric Sp and Rp phosphoramidates.

It will be appreciated that the terms "phosphate" and "phosphate anion" as employed in connection with the present invention include nucleotides and oligonucleotides derived by replacement of one of the oxygen atoms of a naturally occurring phosphate moiety with a heteroatom, an alkyl group or an alkyoxy group. Thus, the terms "phosphate" or "phosphate anion" include naturally occurring nucleotides, phosphodiesters of naturally occurring oligonucleotides, as well as phosphorothioate, alkylphosphonate, phosphotriester, and phosphoamidate oligonucleotides.

Since there exist numerous phosphodiester linkages in an oligonucleotide, substitution of an oxygen atom by another atom such as, for example, sulfur, nitrogen, or carbon in one or more of the phosphate moieties yields a racemic mixture unless such substitution occurs in a stereospecific manner. As a practical matter, see Stec, W.J., Zon, G., and Uznanski, B. (1985), *J. Chromatography,* 326:263, above. Separation of the diastereomers of racemic mixtures of non-stereospecific synthesized oligonucleotides is only possible when there are a minimum of diasymmetric sites, for example, two diasymmetric sites. Since the diasymmetric substituent group at each diastereomeric phosphorus atom could have steric, ionic or other effects on conformation, binding, and the like at each such site, sequence-specific oligonucleotides having all Sp or all Rp chiral phosphorus linkages are desirable.

In accordance with this invention, sequence-specific oligonucleotides are provided comprising substantially pure chiral phosphate linkages such as, for example, phosphorothioate, methylphosphonate, phosphotriester or phosphoramidate linkages. In contrast to prior art synthetic oligonucleotides, at least certain of the chiral phosphorous linkages of the present oligonucleotides are not racemic in nature but, rather, possess relatively high enantiomeric purity. As will be recognized by those skilled in the art, enantiomeric purity -- also known as chiral purity -- is manifested for a chemical compound by the predominance of one enantiomer over the other. Thus, an oligonucleotide can be said to possess a substantially pure chiral phosphate linkage where, for example, the Sp form of that linkage greatly predominates over the Rp form. In accordance with the present invention, at least certain of the chiral phosphate linkages present in an oligonucleotide should have chiral purity greater than about 75%. Preferably such linkages have chiral purity greater than about 90%, more preferably greater than about 95%, even more preferably about 100%. Chiral purity may be determined by any of the many methods known in the art, including but not limited to x-ray diffraction, optical rotary dispersion, and circular dichroism.

The oligonucleotides of the invention are expected to exhibit one or more efficacious properties such as, for example, hybridization with targeted RNA's and DNA's, cellular absorption and transport, or improved enzymatic interaction. At the same time, it is expected that these improvements to the basic oligonucleotide sequences will not significantly diminish existing properties of the basic oligonucleotide sequence. Thus, the present improvements are likely to lead to improved drugs, diagnostics, and research reagents.

Further improvements likely can be effected by making one or more substitutions or modifications to the base or the sugar moieties of the individual nucleosides employed to prepare the chiral oligonucleotides of the invention. Such substitutions or modifications generally comprise derivation at a site on the nucleoside base or at a site on the nucleoside sugar, provided such derivation does not interfere with the stereoselective syntheses of the present invention by, for example, blocking nucleophilic attack of the 5'-phosphate of a first synthon at the 3'-position of a second synthon. In certain embodiments, one or more of the nucleosides of the chiral oligonucleotides of the invention include a naturally occurring nucleoside unit which has been substituted or modified. These non-naturally occurring or "modified" nucleoside units preferably have either structure (4) or structure (5): 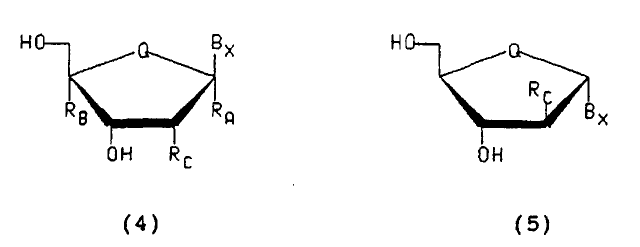 wherein:
Q is O or CHR_{G};
R_{A} and R_{B} are H, lower alkyl, substituted lower alkyl, an RNA cleaving moiety, a group which improves the pharmacokinetic properties of an oligonucleotide, or a group which improves the pharmacodynamic properties of an oligonucleotide;
R_{C} is H, OH, lower alkyl, lower alkenyl, lower alkynyl, substituted lower alkyl, substituted lower alkenyl, substituted lower alkynyl, F, Cl, Br, CN, CF₃, OCF₃, OCN, O-alkyl, O-alkenyl, O-alkynyl, substituted O-alkyl, substituted O-alkenyl, substituted O-alkynyl, S-alkyl, S-alkenyl, S-alkynyl, substituted S-alkyl, substituted S-alkenyl, substituted S-alkynyl, SOMe, SO₂Me, ONO₂, NO₂, N₃, NH₂, NH-alkyl, NH-alkenyl, NH-alkynyl, substituted NH-alkyl, substituted NH-alkenyl, substituted NH-alkynyl, OCH₂CH=CH₂, OCH=CH₂, OCH₂CCH, OCCH, aralkyl, aralkenyl, aralkynyl, heteroaralkyl, heteroaralkenyl, heteroaralkynyl, heterocycloalkyl, poly-alkylamino, substituted silyl, an RNA cleaving moiety, a group which improves the pharmacodynamic properties of an oligonucleotide, or a group which improves the pharmacokinetic properties of an oligonucleotide;
R_{G} is H, lower alkyl, substituted lower alkyl, an RNA cleaving moiety, a group which improves the pharmacokinetic properties of an oligonucleotide, or a group which improves the pharmacodynamic properties of an oligonucleotide; and
B_{X} is a nucleoside base, a blocked nucleoside base, a nucleoside base analog, or a blocked nucleoside base analog.

In preferred embodiments B_{X} is a pyrimidinyl-1 or purinyl-9 moiety as for instance adenine, guanine, hypoxanthine, uracil, thymine, cytosine, 2-aminoadenine or 5-methylcytosine. Other Bₓ groups are shown in U.S. Patent No. 3,687,808 issued August 29, 1972. Preferably, Bₓ is selected such that a modified nucleoside has one of the structures (6)-(11): 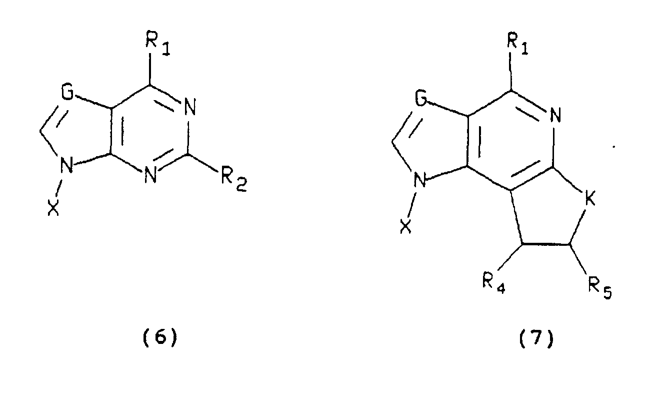 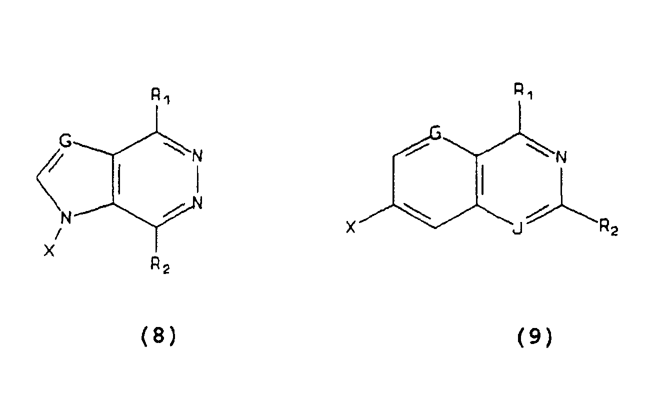 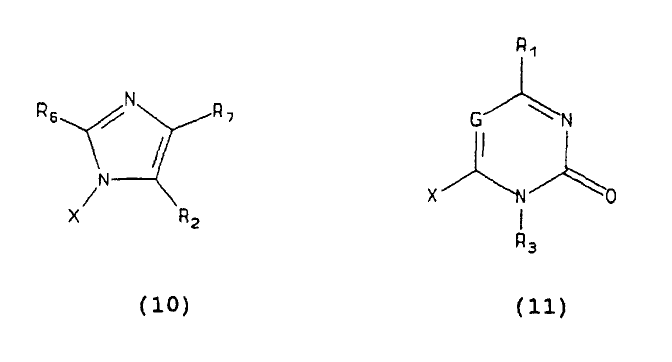 wherein:
G and K are, independently, C or N;
J is N or CR₂R₃;
R₁ is OH or NH₂;
R₂ and R₃ are H, NH, lower alkyl, substituted lower alkyl, lower alkenyl, aralkyl, alkylamino, aralkylamino, substituted alkylamino, heterocycloalkyl, heterocycloalkylamino, aminoalkylamino, hetrocycloalkylamino, polyalkylamino, an RNA cleaving moiety, a group which improves the pharmacokinetic properties of an oligonucleotide, or a group which improves the pharmacodynamic properties of an oligonucleotide;
R₄ and R₅ are, independently, H, OH, NH₂, lower alkyl, substituted lower alkyl, substituted amino, an RNA cleaving moiety, a group which improves the pharmacokinetic properties of an oligonucleotide, or a group which improves the pharmacodynamic properties of an oligonucleotide;
R₆ and R₇ are, independently, H, OH, NH₂, SH, halogen, CONH₂, C(NH)NH₂, C(O)O-alkyl, CSNH₂, CN, C(NH)NHOH, lower alkyl, substituted lower alkyl, substituted amino, an RNA cleaving moiety, a group which improves the pharmacokinetic properties of an oligonucleotide, or a group which improves the pharmacodynamic properties of an oligonucleotide; and
X is a sugar or a sugar substituted with at least one substituent comprising an RNA cleaving moiety, a group which improves the pharmacodynamic properties of an oligonucleotide, or a group which improves the pharmacokinetic properties of an oligonucleotide and other groups as described above for the group R_{C}. It is preferred that X have the general structure (4) or (5).

For the purposes of this invention, improving pharmacodynamic properties means improving oligonucleotide uptake, enhanced oligonucleotide resistance to degradation, and/or strengthened sequence-specific hybridization with RNA and improving pharmacokinetic properties means improved oligonucleotide uptake, distribution, metabolism or excretion. RNA cleaving moieties are chemical compounds or residues which are able to cleave an RNA strand in either a random or, preferably, a sequence-specific fashion.

Exemplary base moieties of the invention are any of the natural pyrimidinyl-1- or purinyl-9- bases including uracil, thymine, cytosine, adenine, guanine, 5-alkylcytosines such as 5-methylcytosine, hypoxanthine, 2-aminoadenine, and other modified bases as depicted in the formulas above. Exemplary sugars include ribofuranosyl, 2'-deoxyribofuranosyl, their corresponding five membered ring carbocyclic analogs as well as other modified sugars depicted in the formulas above. Particularly preferred modified sugars include 2'-fluoro and 2'-O-methyl-2'-deoxyribofuranosyl, i.e. 2'-fluoro and 2'-O-methyl-β-D-erythro-pentofuranosyl.

Lower alkyl groups of the invention include but are not limited to C₁-C₁₂ straight and branched chained, substituted or unsubstituted alkyls such as methyl, ethyl, propyl, butyl, pentyl, hexyl, heptyl, octyl, nonyl, decyl, undecyl, dodecyl, isopropyl, 2-butyl, isobutyl, 2-methylbutyl, isopentyl, 2-methyl-pentyl, 3-methylpentyl, 2-ethylhexyl, 2-propylpentyl. Alkenyl and alkynyl groups include but are not limited to unsaturated moieties derived from the above alkyl groups including but not limited to vinyl, allyl, crotyl, propargyl. Aryl groups include but are not limited to phenyl, tolyl, benzyl, naphthyl, anthracyl, phenanthryl, and xylyl. Halogens include fluorine, chlorine, bromine, and iodine. Suitable heterocyclic groups include but are not limited to imidazole, bis-imidazole, tetrazole, triazole, pyrrolidine, piperidine, piperazine, and morpholine. Carbocyclic groups include 3, 4, 5, and 6-membered substituted and unsubstituted alkyl and alkenyl carbocyclic rings. In some embodiments of the present invention C₁-C₆ lower alkyl, alkenyl and alkynyl groups are preferred. Amines include amines of all of the above alkyl, alkenyl, and aryl groups including primary and secondary amines and "masked amines" such as phthalimide. Amines are also meant to include polyalkylamino compounds and aminoalkylamines such as aminopropylamine and further heterocycloalkylamines such as imidazol-1, 2 or 4-yl-propylamine. Substituent groups for the above include but are not limited to other alkyl, haloalkyl, alkenyl, alkoxy, thioalkoxy, haloalkoxy, and aryl groups as well as halogen, hydroxyl, amino, azido, carboxy, cyano, nitro, mercapto, sulfides, sulfones, and sulfoxides. Other suitable substituent groups also include rhodamines, coumarins, acridones, pyrenes, stilbenes, oxazolo-pryidocarbazoles, anthraquinones, phenanthridines, phenazines, azidobenzenes, psoralens, porphyrins, cholesterols, and other "conjugate" groups.

Sugar derivatizing groups include, but are not limited to H, OH, alkyl, alkenyl, alkynyl, substituted alkyl, substituted alkenyl, substituted alkynyl, F, Cl, Br, CN, CF₃, OCF₃, OCN, O-alkyl, O-alkenyl, O-alkynyl, substituted O-alkyl, substituted O-alkenyl, substituted O-alkynyl, S-alkyl, S-alkenyl, S-alkynyl, substituted S-alkyl, substituted S-alkenyl, substituted S-alkynyl, SOMe, SO₂Me, ONO₂, NO₂, N₃, NH₂, NH-alkyl, NH-alkenyl, NH-alkynyl, substituted NH-alkyl, substituted NH-alkenyl, substituted NH-alkynyl, OCH₂CH=CH₂, OCH=CH₂, OCH₂CCH, OCCH, aralkyl, aralkenyl, aralkynyl, heteroaralkyl, heteroaralkenyl, heteroaralkynyl, heterocycloalkyl, poly-alkylamino, substituted silyl, an RNA cleaving moiety, a group which improves the pharmacodynamic properties of an oligonucleotide, or a group which improves the pharmacokinetic properties of an oligonucleotide.

Methods of synthesizing such modified nucleosides are set forth in copending applications for United States Letters Patent, assigned to the assignee of this invention, and entitled Compositions and Methods for Modulating RNA Activity, serial number 463,358, filed January 11, 1990; Sugar Modified Oligonucleotides That Detect And Modulate Gene Expression, serial number 566,977, filed August 13, 1990; and Compositions and Methods for Modulating RNA Activity, serial number US91/00243, filed January 11, 1991.

The chirally pure phosphorothioate, methylphosphonate, phosphotriester, or phosphoramidate oligonucleotides of the invention may be analyzed by a number of methods. For example, configuration analysis of resulting oligonucleotides having chirally pure phosphate linkages may be determined by the use of [³¹P] NMR chemical shifts. Such chemical shifts have been used to identify the Rp epimer of a phosphorothioate di-nucleotide. See Ludwig, et al. *J. Org*. *Chem*. **1989,** *54*, 631.

The fidelity of sequences of oligonucleotides of the invention can be determined using the sensitivities of heterduplexes to S1 nuclease.

The sequence of oligonucleotides of the invention can be further substantiated by labeling the 3'hydroxyls of phosphorothioate oligonucleotides with [alpha-³²P]cordycepin triphosphate, i.e. 3'-deoxyadenosine-5'-triphosphate. The resultant oligonucleotides may be subjected to enzymatic degradation.

The relative ability of oligonucleotides having substantially chirally pure intersugar linkages to bind to complementary strands is compared by determining the melting temperature of a hybridization complex of an oligonucleotide having substantially chirally pure intersugar linkages and its complementary strand. The melting temperature (Tₘ), a characteristic physical property of double helixes, denotes the temperature in degrees centigrade at which 50% helical versus coiled (unhybridized) forms are present. Tₘ is measured by using the UV spectrum to determine the formation and breakdown (melting) of hybridization. Base stacking, which occurs during hybridization, is accompanied by a reduction in UV absorption (hypochromicity). Consequently a reduction in UV absorption indicates a higher Tₘ. The higher the Tₘ, the greater the strength of the binding of the strands. Non Watson-Crick base pairing has a strong destabilizing effect on the Tₘ. Consequently, as close to optimal fidelity of base pairing as possible is desired to have optimal binding of an oligonucleotide to its targeted RNA.

Oligonucleotides of the invention can also be evaluated as to their resistance to the degradative ability of a variety of exonucleases and endonucleases. Oligonucleotides of the invention may be treated with nucleases and then analyzed, as for instance, by polyacrylamide gel electrophoresis (PAGE) followed by staining with a suitable stain such as Stains All™ (Sigma Chem. Co., St. Louis, MO). Degradation products may be quantitated using laser densitometry.

Fetal calf and human serum can be used to evaluated nucleolytic activity on oligonucleotides having substantially chirally pure intersugar linkages. For instance a oligonucleotide having substantially all-Rp intersugar linkages may be evaluated in this manner. Testing on combinations of 3' or 5' end capped (having one or several phosphate linkages per cap) molecules may be used to establish a combination that yields greatest nuclease stability. Capping can be effected by chemically synthesizing the cap portion of a sequence using purified Rp monomers followed by incorporation of said cap into oligonucleotides on the DNA synthesizer. Analysis involving capping can determine the importance of chirality on nucleolytic stability and the number of linkages required to obtain maximum stability.

The sensitivity of oligonucleotide-RNA heteroduplexes to catalytic activity of RNase H can also be assessed. An oligonucleotide having chirally pure phosphate linkages can be incubated with a radiolabeled target mRNA (synthesized as for instance via T7 RNA polymerase) at various temperatures for hybridization. Heteroduplexes can then be incubated at 37°C with RNase H from *E. coli* according to the procedure of Minshull, J. and Hunt, T., *Nuc*. *Acid Res.,* 6433-6451 (1986). Products may then be assessed for RNase H activity by Northern Blot analysis wherein products are electrophoresed on a 1.2% agarose/formaldehyde gel and transferred to nitrocellulose. Filters may then be probed using a random primer [³²P]-labeled cDNA complementary to target mRNA and quantitated by autoradiography. Comparisons between different oligonucleotides can be made to determine the impact of chirality on the ability to act as a substrate for RNase H when complexed to RNA.

Comparisons of the susceptibility of heteroduplexes to the catalytic action of *E*. *coli* RNase H and mammalian RNAse H can be performed. Heteroduplexes can be incubated in rabbit reticulocyte lysates under conditions of translation and assayed via Northern blot analysis for catalytic cleavage of mRNA by endogenous RNase H. This allows for determination of the effects of chirality on mammalian RNAse H activity.

Oligonucleotides having substantially chirally pure intersugar linkages can also be evaluated for inhibition of gene expression in cell culture model systems. To determine if an oligonucleotide having substantially pure chirally pure intersugar linkages is more potent or a more specific inhibitor of gene expression, an oligonucleotide having substantially chirally pure intersugar linkages designed to target reporter genes may be synthesized and tested in cell culture models of gene expression. The use of the vector pSV2CAT has previously been described to measure antisense effects on gene expression; see Henthorn, P., Zervos, P., Raducha, M., Harris, H., and Kadesch, T., *Proc.Natl.Acad.Sci.USA*, 85:6342-6346 (1988). This vector contains the bacterial chloramphenicol acetyl transferase gene under regulatory controls of the SV40 promoter. Utilizing a 15-mer oligonucleotide having all-Rp intersugar linkages of a sequence complementary to the initiation of translation of the CAT mRNA, pSV2CAT may be transfected into HeLa cells and, following treatment of the cells for 48 hr with an oligonucleotide having all-Rp intersugar linkages, CAT activity may then be assayed in the cells. The activity of an oligonucleotide having substantially chirally pure intersugar linkages in inhibition of gene expression may then be compared directly with a chemically synthesized random oligonucleotide having diastereomeric intersugar linkages and natural phosphodiester oligonucleotides of the same sequence.

The vector pSV2APAP, see Marcus-Sekura, C.J., Woerner, A.M., Shinozukea, K., Zon,G., Quinnan, G.V. Jr., *Nucleic Acids Research,* 15:5749-5763 (1987), contains the mammalian placental alkaline phosphatase gene (PAP). This can also be used as a reporter for measuring antisense effects on gene expression. PAP has advantages over CAT as a reporter gene in that it is a mammalian gene, rather than a bacterial gene that contains introns and other RNA processing signals. It is presently believed that PAP expression mimics more closely the events in natural mammalian gene expression. A 15-mer oligonucleotide having substantially chirally pure intersugar linkages as described above for the CAT mRNA can be examined in parallel with a chemically synthesized racemic oligonucleotide and a natural phosphodiester oligonucleotide having similar sequences. The PAP and CAT reporter constructs are used as controls in reciprocal experiments to test for non-specific effects on gene expression.

The chirally pure phosphorothioate, methylphosphonate, phosphotriester or phosphoramidate oligonucleotides of the invention can be evaluated for their ability to act as inhibitors of RNA translation in vivo. Various therapeutic areas can be targeted for such antisense potential. These therapeutic areas include but are not limited to herpes virus (HSV), the TAR and tat regions of HIV, the codon regions of *Candida albicans* chitin synthetase and *Candida albicans* β tubulin, papilloma virus (HPV), the ras oncogene and protooncogene, ICAM-1 (intercellular adhesion molecule-1) cytokine, and 5'-lipoxygenase. A targeted region for HSV includes GTC CGC GTC CAT GTC GGC (SEQ ID NO:1). A targeted region for HIV includes GCT CCC AGG CTC AGA TCT (SEQ ID NO:2). A targeted region for *Candida albicans* includes TGT CGA TAA TAT TAC CA (SEQ ID NO:3). A targeted region for human papillomavirus, e.g. virus types HPV-11 and HPV-18, includes TTG CTT CCA TCT TCC TCG TC (SEQ ID NO:4). A targeted region for ras includes TCC GTC ATC GCT CCT CAG GG (SEQ ID NO:5). A targeted region for ICAM-1 includes TGG GAG CCA TAG CGA GGC (SEQ ID NO:6) and the sequence CGA CTA TGC AAG TAC (SEQ ID NO:9) is a useful target sequence for 5-lipoxygenase. In each of the above sequences the individual nucleotide units of the oligonucleotides are listed in a 5' to 3' sense from left to right.

The phosphorothioate, methylphosphonate, phosphotriester or phosphoramidate oligonucleotides of the invention may be used in therapeutics, as diagnostics, and for research, as specified in the following copending applications for United States Letters Patent assigned to the assignee of this invention: Compositions and Methods for Detecting and Modulating RNA Activity, Serial No. 463,358, filed January 11, 1990; Antisense Oligonucleotide Inhibitors of Papilloma Virus, Serial No. 445,196 Filed 12/4/89; Oligonucleotide Therapies for Modulating the Effects of Herpesvirus, US 5,248,670 Serial No. 485,297, filed 2/26/90; Reagents and Methods for Modulating Gene Expression Through RNA Mimicry Serial No. 497,090, filed 3/21/90; Oligonucleotide Modulation of Lipid Metabolism, Serial No. 516,969, filed 4/30/90; Oligonucleotides for Modulating the Effects of Cytomegalovirus Infections, Serial No. 568,366, filed August 16, 1990; Antisense Inhibitors of the Human Immunodeficiency Virus US 5,166,195 Serial No. 521,907, filed 5/11/90; Nuclease Resistant Pyrimidine Modified Oligonucleotides for Modulation of Gene Expression, Serial No.558,806, filed 7/27/90; Novel Polyamine Conjugated Oligonucleotides US 5,138,045 Serial No. 558,663, filed 7/27/90; Modulation of Gene Expression Through Interference with RNA Secondary Structure, Serial No. 518,929, filed 5/4/90; Oligonucleotide Modulation of Cell Adhesion, Serial No. 567,286, filed 8/14/90; Inhibition of Influenza Viruses, Serial No. 567,287, filed 8/14/90; Inhibition of *Candida,* Serial No. 568,672, filed 8/16/90; and Antisense Oligonucleotide Inhibitors of Papillomavirus WO 91/08313 Serial No. PCT/US90/07067, filed 12/3/90. These patents disclose a number of means whereby improved modulation of RNA and DNA activity may be accomplished through oligonucleotide interaction.

The oligonucleotides of the invention may be prepared by enzymatic methods. Ribonucleosides (NTPαS) and 2'-deoxyribonucleosides (dNTPαS) such as 5'-O-(thiotriphosphates) have been synthesized as Sp and Rp racemic mixtures such as by using the methodology of Ludwig and Eckstein; Ludwig, et al., *J*. *Org*. *Chem*. **1989,** *54*, 631-635. In this exemplary synthetic scheme, unprotected nucleosides can be reacted with 2-chloro-4H-1,3,2-benzodioxaphosphrin-4-one, which phosphitylates the 5'-hydroxyl group. Subsequent reaction with pyrophosphate yields cyclic triphosphate derivatives which are reactive to sulfur, yielding mixtures of Rp and Sp nucleoside 5'-O-(1-thiotriphosphates), i.e. alpha-thiotriphosphates. The products can be purified such as by using DEAE-Sephadex chromatography and identified with NMR spectroscopy by characteristic Rp or Sp chemical shifts.

As is shown in the examples below, pure Rp and Sp nucleosides 5'-O-(1-thiotriphosphates) diastereomers can be readily isolated on a preparative scale using, for example, reverse phase HPLC chromatography. Such HPLC isolated nucleotide diastereomers can be further characterized by analytical HPLC comparisons with commercial samples of such Rp and Sp nucleoside 5'-O-(1-thiotriphosphates) diastereomers.

Enzymatic synthesis of sequence specific natural oligonucleotides, i.e. natural phosphodiester oligonucleotides, can be effected by the use of an appropriate nuclease in the presence of a template and primer. In a like manner racemic mixtures of oligonucleotides having chirally mixed intersugar linkages can be synthesized. According to the teachings of the present invention, such enzymatic synthesis can also be expanded to include the synthesis of sequence specific oligonucleotides having substantially chirally pure intersugar linkages by utilizing enantiomerically pure all-Sp or all-Rp nucleosides such as nucleoside 5'-O-(1-thiotriphosphates) as substrates for appropriate nucleases in the presence of a sequence specific template and a primer. The nucleosides may be naturally occuring or, to the extent that they are suitable substrates for nucleases, such nucleosides may have modified bases or sugars.

For example, a commercially available DNA polymerase Sequenase^{Tm} (U.S. Biochemical, Inc., Cleveland, OH) may be used to synthesize enantiomerically pure oligonucleotides having chiral phosphate linkages using a phosphodiester oligonucleotide template and a racemic phosphorous linkage oligonucleotide primer. Using this polymerase both phosphodiester and chiral phosphate linkage primers may be extended.

Yields of enzymatically synthesized oligonucleotides having chiral phosphate linkages can be optimized by repetitive additions of template and primer, by repetitive additions of polymerase, by repetitive additions of nucleoside triphosphates or by combinations of some or all of these. For instance, repetitive additions of template and primer results in maximizing yields via an enzymatic cascade. Further optimization can be achieved by pre-hybridization of template and primer together in system buffer, followed by cooling and addition of nucleoside triphosphates and polymerase.

Suitable polymerase may be selected to yield either DNA or RNA oligonucleotides having chiral phosphorous linkages. Such polymerases include but are not necessarily limited to T7 DNA polymerase, modified T7 DNA polymerases such as the above referenced Sequenase, *E*. *coli* DNA polymerase, DNA poly Klenow fragment polymerase, M. luteus polymerase, T4 bacteriophage polymerase, modified T4 DNA polymerase, T7 RNA polymerase and *E*. *coli* RNA polymerase.

The enzymatic synthesis proceeds with inversion about the chiral center of the phosphorus atom. For example, the use of all-Sp alpha-thiotriphosphates yields substantially all Rp phosphorothioate oligonucleotides while use of all-Rp alpha-thiotriphosphates yields substantially all Sp phosphorothioate oligonucleotides. Alternatively oligonucleotides having chiral phosphate linkages such as phosphorothioate oligonucleotides may be synthesized from Sp-Rp racemic mixtures of nucleoside, such as 5'-O-(1-thiotriphosphates) utilizing metal ions in reaction solutions to promote preferential incorporation of one or the other of the chiral alpha-S-triphosphates. As noted above polymerase synthesis of such phosphorothioate oligonucleotide is accomplished with inversion about the chiral center of the precursor nucleoside alpha-S-triphosphate. While not wishing to be bound by theory, it is believed that optimization of an all-Rp configuration may be accomplished by addition of a (relative) high concentration of magnesium ion in the reaction buffer utilizing for instance an *E. coli* polymerase. In a like manner, again while we do not wish to be bound by theory, an all-Sp configuration might be obtained by utilizing a (relative) high manganese ion concentration in the reaction buffer.

Radiolabeling can be used to assist with identification of oligonucleotides having substantially chirally pure intersugar linkages. For DNA synthesizer synthesized racemate phosphorothioate oligonucleotides, [³⁵S] (radiolabeled elemental sulfur) can be used for oxidation of the hydrogenphosphonate oligomers obtained from the DNA synthesizer. Labeling of enzymatic synthesized phosphorothioate oligomers can be accomplished such as with [alpha-³²P]ATP and ligase or [alpha-³⁵S]ATPs in the polymerase chain raction. Also, radiolabeled nucleoside triphosphates can be used in probe and sequencing analysis. Autoradiograms are prepared in standard manners.

Templates of the present invention are most preferably areas of nucleic acid sequence which direct synthesis of disease potentiating proteins. Short oligonucleotides that base pair to a region of said template oligonucleotide act as primers which form the starting point for oligonucleotide synthesis by polymerases.

Oligonucleotides having substantially chirally pure phosphate linkages may be synthesized using a primer which may be selected to have a site thereon that is susceptible to nuclease cleavage, as for example restriction endonuclease cleavage. Said cleavage site may be located at the 3' end of said primer. Cleavage at said site by an appropriate restriction endonuclease results in oligonucleotides deriving a first 5' end nucleoside from said primer. Additional nucleosides of said oligonucleotides of the present invention are those nucleoside added via enzymatic methods.

By selecting appropriate restriction nucleases in conjuction with selected primers, various 5'-terminal nucleosides of desired oligonucleotides are appropriately positioned at the 5' end of an oligonucleotide. Thus, any endonuclease recognition site can be designed as long as the staggered cut results in one nucleoside from the primer being the first 5' nucleoside of the newly synthesized sequence specific oligonucleotide of the invention. This results in the generation of different nucleosides on 5' ends of enzymatically synthesized oligonucleotides of the invention.

Upon completion of enzymatic extension of said primer on an appropriate template of a desired sequence, oligonucleotides of the invention may be released from said primer by use of appropriate nuclease. For example, for incorporation of a guanosine nucleoside at the 5' end of desired oligonucleotides, a primer having an CTGCAG sequence at its 3' terminal end may be used. Use of a Pst 1 restriction nuclease then may cleave the A-G linkage. The guanosine nucleoside component of this A-G linkage may thus incorporated as a 5' terminal nucleoside of desired oligonucleotides. Other restriction endonuclease include but are not limited to BamH1, Smal and HinD III restriction endonucleases.

Oligonucleotides still associated with said template may be disassociated from said template and then purified, for instance by gel electrophoresis and/or chromatography. For example, suitable purification can be accomplished utilizing standard polyacrylamide/urea gel electrophoresis coupled with SepPac (Millipore, Miford, MA) chromatography. Other useful chromatographic techniques include HPLC chromatography.

The oligonucleotides of the invention may also be preferably prepared via the process shown in Scheme 1, wherein a selected nucleotide is coupled to another nucleotide or to a growing oligonucleotide chain via a nucleophilic displacement reaction. As will be recognized, this process is also applicable to the preparation of oligonucleotides comprising non-chiral phosphodiester linkages. In Scheme 1, R_{F} is a phosphate blocking group, B_{X} is a suitable heterocyclic nucleoside base (blocked or unblocked), R_{X} is a sugar derivatizing group and Q is an oxygen or methylene group. Together R_{D} and R_{E} are the necessary oxygen, sulfur, nitrogen, substituted nitrogen, alkoxy or thioalkoxy groups that form the phosphorothioate, methylphosphonate, phosphotriester or phosphoramidate linking groups. For Scheme 1, the Y group of the above formulas is depicted as a CPG (Controlled Pore Glass) group. Other Y groups also may be used.

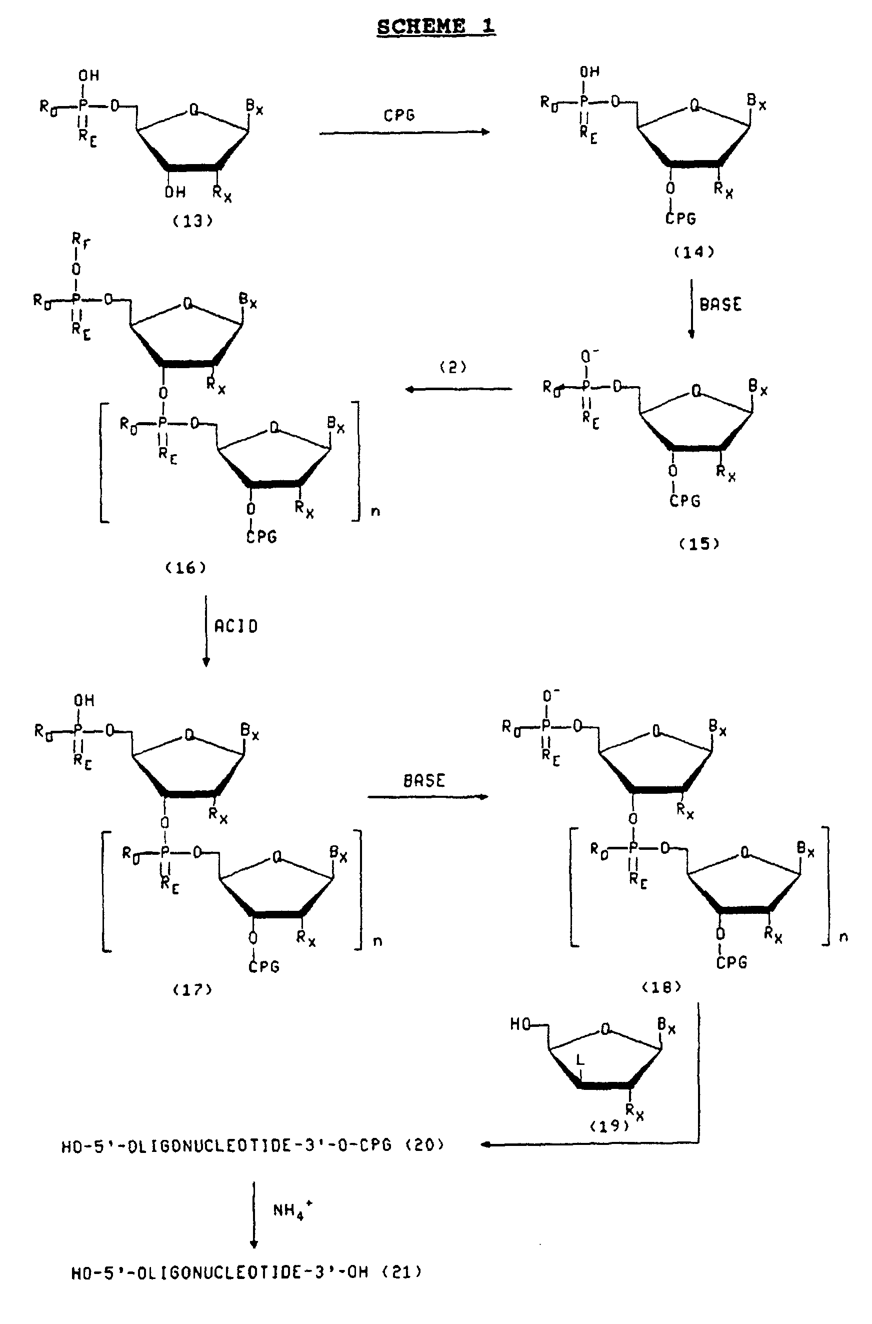

In Scheme 1, a first nucleotide (13) is attached to a solid state CPG support via its 3'-hydroxyl group in a standard manner, yielding compound (14). Compound (14), which forms a first synthon, is treated with an appropriate base, producing anionic compound (15). Compound (15) is reacted with a first unit of compound (2) -- a xylofuranosyl nucleotide bearing blocking group R_{F} on its phosphate functionality and leaving group L at its 3' position. Compound (2) is a second synthon. The B_{X} moiety of structure (2) may be the same as or different than the B_{X} moiety of compound (15), depending on the sequence of the desired oligonucleotide. The anion of compound (15) displaces the leaving group at the 3' position of compound (2). The displacement proceeds via inversion at the 3' position of the second synthon, forming compound (16) with n = 1.

The resulting phosphorothioate, methylphosphonate, phosphotriester or phosphoramidate linkage of compound (16) extends from the 5' position of the first synthon (compound (14)) to the 3' position of the second synthon (compound (2)). The inversion at the 3' position of the second synthon results in the final configuration at the 3' nucleotide derived from the second synthon being a normal ribofuranosyl sugar conformation. Compound (16) on the solid state CPG support is now washed to free it of any unreacted compound (2).

The second synthon carries a phosphate blocking group R_{F} on its phosphorothioate, methylphosphonate, phosphotriester or phosphoramidate phosphate group. After coupling of the second synthon to the first synthon to yield compound (16) wherein n = 1 and washing, the phosphate blocking group R_{F} is removed with an acid, yielding compound (17) wherein n = 1. Compound (17), which represents a new first synthon, is now treated with base to generate a further anionic, compound (18) with n = 1. Compound (18) is suitable for nucleophilic attack on a further unit of compound (2) (the second synthon) to form a new compound (16) wherein n = 2. In this further unit having compound (2), the B_{X} moiety may be the same or different from the B_{X} moiety of either of the nucleotides of compound (16) wherein n = 1, depending on the desired sequence.

Compound (16) wherein n = 2 is washed and then treated with acid to deblock the R_{F} blocking group, yielding a further new first synthon, compound (17) wherein n = 2. This new first synthon, is now ready to be further cycled by treatment with base to yield compound (18) wherein n = 2, which is now reacted with a further unit having compound (2) to yield a further unit of having structure (16) wherein n = 3. Again, B_{X} may be the same or different than previously B_{X} moieties. The cycle is repeated for as many times as necessary to introduced further nucleotides of the desired sequence via compound (2).

If it is desired to have the 5' terminal end of the final oligonucleotide as a phosphate group, then the last compound (17) is appropriately removed from the CPG support. If it is desired to have the 5' terminal end as a hydroxyl group, then the penultimate nucleotide is added as compound (22), it is converted to compounds (16), (17), and (18) and the resulting compound (18) is reacted with a xylofuranosyl nucleoside, compound (19). Compound (19), like compound (2), includes a leaving group L within its structure. Reaction of compound (19) with compound (18) yields oligonucleotide, compound (20), which is released from the CPG support with concentrated ammonium ion to yield the desired oligonucleotide, compound (21). The ammonium ion treatment will also remove any base blocking groups as is standard in automated oligonucleotide synthesis.

In summary, as shown in Scheme 1, a phosphorothioate, methylphosphonate, phosphotriester or phosphoramidate 5' nucleotide (or the 5'-terminal nucleotide of a growing oligonucleotide) functions as a first synthon. This is converted to an anion with a base. This anion displaces a leaving group at the 3' position of a xylofuranosyl nucleotide. The xylofuranosyl nucleotide comprises a second synthon. The displacement proceeds via inversion at the 3' position of the second synthon with the resulting phosphorothioate, methylphosphonate, phosphotriester or phosphoramidate linkage that is formed extending from the 5' position of the first synthon to the 3' position of the second synthon. The inversion at the 3' position of the second synthon results in the final configuration at the 3' nucleotide derived from the second synthon being a normal ribofuranosyl sugar conformation. It has a 3' to 4' trans orientations (a ribofuranosyl sugar conformation) that is identical to natural or wild type oligonucleotides.

The second synthon carries a phosphate blocking group on its phosphorothioate, methylphosphonate, phosphotriester or phosphoramidate phosphorus group. After coupling of the second synthon to the first synthon, this phosphate blocking group is removed, generating a new first synthon having an anion at its 5' phosphate suitable for nucleophilic attack on a further second synthon. Thus, after coupling of the first and second synthon, the newly joined first and second synthons now form a new first synthon. The oligonucleotide is elongated nucleotide by nucleotide via the nucleophilic attack of a phosphate anion at the 5' end of the growing oligonucleotide chain on the leaving group at the 3' position of the soon-to-be-added xylofuranosyl configured second synthon nucleotide.

It is presently preferred that the phosphate blocking group be a base stable, acid labile group. Such a phosphate blocking group maintains the phosphate moiety of the second synthon in a protected form that cannot react with the leaving group of the second synthon. This inhibits polymerization of the second synthon during the coupling reaction.

The nucleophilic coupling of the first and second synthons is a stereoselective coupling process that maintains the stereospecific configuration about the phosphorus atom of the first synthon. Thus the particular Sp or Rp diastereomeric configuration of a resolved phosphorothioate, methylphosphonate, phosphotriester or phosphoramidate moiety at the 5' end of a starting second synthon nucleotide or the 5' terminal end of a growing first synthon oligonucleotide is maintained. While the sugar portion of the second synthon undergoes inversion about its 3' position as a result of the coupling process, the phosphate portion of the second synthon retains its stereospecific configuration. After coupling of the second synthon to the first, the phosphate moiety (for example, the phosphorothioate, alkylphosphate, phosphoamidate or phosphotriester moiety) of the second synthon retains its original stereochemistry. That is, an Rp diastereomeric second synthon retains the Rp configuration of its phosphate moiety, while an Sp diastereomeric second synthon retains the Sp configuration of its phosphate moiety.

For example, to form an Rp chiral phosphorothioate, methylphosphonate, phosphotriester or phosphoramidate oligonucleotide a first Rp diastereomeric nucleotide is chosen as the first synthon. It is coupled with an Rp diastereomeric second synthon nucleotide. The resulting dinucleotide maintains the Rp orientation at the inter-nucleotide linkage between the first and second nucleotides. When a third nucleotide is next coupled to the first two, the Rp diastereomeric phosphate moiety from the second nucleotide forms the inter-nucleotide linkage between the second and third nucleotides and is maintained in the Rp orientation. If the third nucleotide was also an Rp diastereomeric nucleotide then when a fourth nucleotide is added to the growing oligonucleotide chain, the inter-nucleotide linkage between nucleotides three and four is also an Rp diastereomeric linkage. If each added "second synthon" is also an Rp diastereomer, then the resulting oligonucleotide will contain only Rp inter-nucleotide linkages. If an oligonucleotide having Sp inter-nucleotide linkages is desired, then the first nucleotide and each of the added subsequent nucleotides are selected as Sp diastereomeric nucleotides.

The first synthon can be a first nucleotide or a growing oligonucleotide chain. If it is desired that each of the nucleotides of the oligonucleotide be ribofuranoside configured nucleotides, then the first nucleotide is selected as a ribofuranoside configured nucleotide. Each added second synthon, while added as a xylofuranoside configured nucleotide, after inversion is converted to a ribofuranoside configured nucleotide.

The 3' position of the first nucleotide is either blocked if a solution reaction is practiced or is coupled to a solid state support if a solid state reaction (as for instance one utilizing a DNA synthesizer) is practiced. Each additional nucleotide of the oligonucleotide is then derived from a xylofuranosyl nucleotide, i.e. a second synthon. Because the first nucleotide of the oligonucleotide can be a "standard" ribofuranosyl nucleotide coupled via its 3' hydroxyl to a solid state support, the standard solid state supports known in the art, such as controlled pore glass (CPG) supports, can be utilized and the second synthons added as reagents to the growing oligonucleotide on a standard DNA synthesizer such as, for example, an Applied Biosystems Inc. 380B Nucleic Acid Synthesizer. However, unlike standard DNA synthesizer techniques, nucleotide coupling is not achieved using activated phosphoamidate chemistry. Instead, the above-noted nucleophilic displacement reaction of a phosphate anion on a 3' leaving group of a xylofuranosyl nucleotide is utilized as the coupling reaction. Even when chiral phosphoramidate phosphate linkages are being incorporated into the sequence-specific chiral oligonucleotides of the invention, the phosphoramidate groups of individual nucleotides are not directly used to effect the coupling reaction between nucleotides. Rather, as with phosphorothioates, alkylphosphonates, and phosphotriesters, an anion is generated at the phosphate moiety. It is this anion -- not an activated amidate species -- that is the activated species for effecting coupling.

Once the first nucleotide is loaded on a solid state support utilizing standard techniques, the anion necessary for nucleophilic attack is generated via treatment of the first nucleotide, i.e. the first synthon, with a base. Suitable bases include but are not limited to sodium hydride, methylmagnesium chloride, and t-butylmagnesium chloride. These are used in a suitable solvent such as acetonitrile, tetrahydrofuran or dioxane.

The second synthon is added either concurrently with the base or subsequent to it. After coupling, the growing oligonucleotide is washed with a solvent and then treated with a reagent to effect deblocking of the phosphate blocking group of the second synthon. If a preferred acid-labile blocking group is used to block the phosphate of the second synthon, deblocking is easily effected by treating the growing oligonucleotide on the solid state support with an appropriate acid.

Suitable acid-labile blocking groups for the phosphates of the second synthon include but are not limited to t-butyl, dimethoxytrityl (DMT) or tetrahydropyranyl groups. Suitable acids for deblocking the second synthon phosphate blocking group include but are not limited to acetic acid, trichloroacetic acid, and trifluoromethane sulfonic acid. Such acids are suitably soluble in solvents such as tetrahydrofuran, acetonitrile, dioxane, and the like.

Following treatment with an appropriate deblocking reagent to effect deblocking of the phosphate protecting group, the growing oligonucleotide is then washed with an appropriate solvent such as tetrahydrofuran, acetonitrile or dioxane. The oligonucleotide is now ready for the addition of a further nucleotide via treatment with base to generate an anion on the 5' terminal phosphate followed by the addition of a further second synthon. Alternatively, the anion can be generated concurrently with addition of a further second synthon. Suitable leaving groups for inclusion at the 3' position of the xylofuranosyl second synthon include but are not limited to the group consisting of halogen, alkylsulfonyl, substituted alkylsulfonyl, arylsulfonyl, substituted arylsulfonyl, hetercyclcosulfonyl or trichloroacetimidate. A more preferred group includes chloro, fluoro, bromo, iodo, p-(2,4-dinitroanilino)benzenesulfonyl, benzenesulfonyl, methylsulfonyl (mesylate), p-methylbenzenesulfonyl (tosylate), p-bromobenzenesulfonyl, trifluoromethylsulfonyl (triflate), trichloroacetimidate,acyloxy,2,2,2-trifluoroethanesulfonyl, imidazolesulfonyl, and 2,4,6-trichlorophenyl. These leaving groups are subject to SN₂ displacement reactions with inversion about the 3' position of the sugar to provide the required 3'-4' trans ribofuranosyl configuration after inversion. The ionized oxygen atom of the phosphate moiety of the first synthon displaces these leaving groups to effect the coupling of the first synthon to the second synthon.

A further class of leaving groups include certain sugar-base cyclonucleosides. These include 2,3' or 6,3'-cyclopyrimidines and 8,3'-cyclopurine nucleosides. These nucleosides are alternately known as anhydro nucleosides. Since the sugar-heterocycle bond of such cyclonucleosides is "syn" with the heterocycle base, nucleophilic addition with inversion at this site also yields the desired ribofuranoside configuration of the added nucleotide after addition of the second synthon to the first synthon. The linking atom between the 3' position of the sugar and the 2 or 6 position of a pyrimidine base or the 3' position of the sugar and the 8 position of the purine base can be oxygen, sulfur or nitrogen.

Since a basic environment is created during coupling of the first synthon to the second synthon and an acidic environment (utilizing the preferred acid-labile phosphate blocking group) is created during deblocking of the phosphate blocking group from the nucleotide derived from the second synthon, if blocking groups are utilized on the base or sugar portions of the nucleotides such base or sugar blocking groups must be stable to both acidic and basis conditions. Suitable blocking groups for the heterocyclic base or the sugar are selected to be stable to these conditions. One type of blocking groups that can be used are acid\base stable, hydrogenolysis-sensitive blocking groups; that is, blocking groups which can be removed with molecular hydrogen but not with acid or base. A benzyl blocking group is such a suitable hydrogenolysis-sensitive blocking group.

Other heterocycle base or sugar blocking groups are those that require more pronounced acid or base treatment to de-block than may be experienced during the basic activation of the nucleophilic displacement reaction of the second synthon blocking group or the acidic removal of the phosphate blocking group. Two such blocking groups are the benzoyl and isobutyryl groups. Both of these require strong basic conditions for their removal. These basic conditions are more stringent than that required to generate the phosphate anion for the nucleophilic displacement reaction. This allows the use of such benzoyl or isobutyryl blocking groups for the 6-amino group of adenine, the 2-amino group of guanine, and the 4-amino group of cytosine. Suitable precursor molecules for the second synthons include the xylo derivatives of the common nucleosides. Certain of these "xylo nucleosides" are commercially available and others are known in the nucleoside literature.

Xylo nucleosides include but are not limited to xylo derivatives of adenosine, guanosine, inosine, uridine, cytidine, thymidine, 5-methylcytidine, and 2-aminoadenosine, i.e. 9-(β-D-xylofuranosyl)adenine, 9-(β-D-xylofuranosyl)guanine, 9-(β-D-xylofuranosyl)hypoxanthine, 1-(β-D-xylofuranosyl)uracil, 1-(β-D-xylofuranosyl)cytosine, 1-(β-D-xylofuranosyl)thymine, 5-methyl-1-(β-D-xylofuranosyl)cytosine, and 2-amino-9-(β-D-xylofuranosyl)adenine. They also include the xylo equivalents of the common 2'-deoxy nucleosides such as 9-(β-D-2'-deoxy-threo-pentofuranosyl)adenine, 9-(β-D-2'-deoxy-threo-pentofuranosyl)guanine, 9-(β-D-2'-deoxy-threo-pentofuranosyl)hypoxanthine, 1-(β-D-2'-deoxy-threo-pentofuranosyl)uracil, 1-(β-D-2'-deoxy-threo-pentofuranosyl)cytosine, 1-(β-D-2'-deoxy-threo-pentofuranosyl)thymine,5-methyl-1-(β-D-2'-deoxy-threo-pentofuranosyl)cytosine, and 2-amino-9-(β-D-2'-deoxy-threo-pentofuranosyl)adenine.

Other preferred nucleosides that are suitable precursors for the second synthon include but are not limited to 2'-fluoro, 2'-methoxy, 2'-O-allyl, 2'-methyl, 2'-ethyl, 2'-propyl, 2'-chloro, 2'-iodo, 2'-bromo, 2'-amino, 2'-azido, 2'-O-methyl, 2'-O-ethyl, 2'-O-propyl, 2'-O-nonyl, 2'-O-pentyl, 2'-O-benzyl, 2'-O-butyl, 2'-O-(propylphthalimide), 2'-S-methyl, 2'-S-ethyl, 2'-aminononyl, 2'-aralkyl, and 2'-alkylheterocyclo such as propylimidazoyl derivatives of the above 2'-deoxy-threo-pentofuranosyl nucleosides. Representatives of this group include but are not limited to 9-(β-D-2'-deoxy-2'-fluoro-threo-pentofuranosyl)adenine, 9-(β-D-2'-deoxy-2'-fluoro-threo-pentofuranosyl)guanine, 9-(β-D-2'-deoxy-2-fluoro-threo-pentofuranosyl)hypoxanthine, 1-(β-D-2'-deoxy-2'-fluoro-threo-pentofuranosyl)uracil, 1-(β-D-2'-deoxy-2'-fluoro-threo-pentofuranosyl)cytosine, 1-(β-D-2'-deoxy-2'-fluoro-threo-pentofuranosyl)thymine, 5-methyl-1-(β-D-2'-deoxy-2'-fluoro-threo-pentofuranosyl)cytosine, 2-amino-9-(β-D-2'-deoxy-2'-fluoro-threo-pentofuranosyl)adenine, 9-(β-D-2'-deoxy-2'-methoxy-threo-pentofuranosyl)adenine, 9-(β-D-2'-deoxy-2'-methoxy-threo-pentofuranosyl)guanine, 9-(β-D-2'-deoxy-2-methoxy-threo-pentofuranosyl)hypoxanthine, 1-(β-D-2'-deoxy-2'-methoxy-threo-pentofuranosyl)uracil, 1-(β-D-2'-deoxy-2'-methoxy-threo-pentofuranosyl)cytosine, 1-(β-D-2'-deoxy-2'-methoxy-threo-pentofuranosyl)thymine, 5-methyl-1-(β-D-2'-deoxy-2'-methoxy-threo-pentofuranosyl)cytosine,2-amino-9-(β-D-2'-deoxy-2'-methoxy-threo-pentofuranosyl)adenine, 9-(β-D-2'-deoxy-2'-O-allyl-threo-pentofuranosyl)adenine, 9-(β-D-2'-deoxy-2'-O-allyl-threo-pentofuranosyl)guanine, 9-(β-D-2'-deoxy-2'-O-allyl-threo-pentofuranosyl)hypoxanthine, 1-(β-D-2'-deoxy-2'-O-allyl-threo-pentofuranosyl)uracil, 1-(β-D-2'-deoxy-2'-O-allyl-threo-pentofuranosyl)cytosine,1-(β-D-2'-deoxy-2'-O-allyl-threo-pentofuranosyl)thymine, 5-methyl-1-(β-D-2'-deoxy-2'-O-allyl-threo-pentofuranosyl)cytosine,2-amino-9-(β-D-2'-deoxy-2'-O-allyl-threo-pentofuranosyl)adenine, 9-(β-D-2'-deoxy-2'-methyl-threo-pentofuranosyl)adenine, 9-(β-D-2'-deoxy-2'-chloro-threo-pentofuranosyl)guanine, 9-(β-D-2'-deoxy-2-amino-threo-pentofuranosyl)hypoxanthine, 1-(β-D-2'-deoxy-2'-O-nonyl-threo-pentofuranosyl)uracil, 1-(β-D-2'-deoxy-2'-O-benzyl-threo-pentofuranosyl)cytosine, 1-(β-D-2'-deoxy-2'-bromo-threo-pentofuranosyl)thymine,5-methyl-1-(β-D-2'-deoxy-2'-O-butyl-threo-pentofuranosyl)cytosine, and 2-amino-9-[β-D-2'-deoxy-2'-O-(propylphthalimide)-threo-pentofuranosyl)adenine. The 2'-deoxy-2'-fluoro-threo-pentofuranosyl, and 2'-deoxy-2'-methoxy-threo-pentofuranosyl nucleosides are particularly preferred in that the 2'-fluoro and 2'-methoxy groups give improved nuclease resistance to oligonucleotide bearing these substituents on their respective nucleotides.

Further preferred nucleosides that are suitable precursors for the second synthon include but are not limited to the xylofuranosyl or 2'-deoxy-threo-pentofuranosyl derivatives of 3-deaza purine and 2-substituted amino purine nucleosides including but not limited to 3-deaza-2'-deoxyguanosine, 3-deaza-3-nonyl-2'-deoxyguanosine, 3-deaza-3-allyl-2'-deoxyguanosine, 3-deaza-3-benzyl-2'-deoxyguanosine, 3-deaza-3-nonyl-2'-deoxyguanosine, N2-[imidazol-1-yl-(propyl)]-2'-deoxyguanosine, and 2-amino-N2-[imidazol-1-yl(propyl)]adenosine.

Another preferred group of nucleoside precursors for the second synthon include the carbocyclic nucleosides, i.e. nucleosides having a methylene group in place of the pentofuranosyl ring oxygen atom. Such carbocyclic compounds may exhibit increased stability towards chemical manipulation during activation of the xylo nucleosides for nucleophilic attack.

The xylo nucleoside or derivatized xylo nucleoside is reacted with a suitable phosphorylating agent to phosphorylate the second synthon precursor. Various phosphorylation reactions are known in the art such as those described in *Nucleotide Analogs,* by Karl Heinz Scheit, John Wiley & Sons, 1980, Chapter Four - Nucleotides with Modified Phosphate Groups and Chapter Six - Methods Of Phosphorylation; Conjugates Of Oligonucleotides and Modified Oligonucleotides: A Review Of Their Synthesis and Properties, Goodchild, J. (1990), *Bioconjugate Chemistry,* 1:165; and Antisense Oligonucleotides: A New Therapeutic Principle, Uhlmann, E. and Peyman, A. (1990), *Chemical Reviews,* 90:543.

Preferred phosphorylating agents include phosphoryl chlorides. Suitable phosphoryl chlorides include but are not limited to thiophosphoryl chloride, t-butoxyphosphoryl chloride, t-butoxy(methyl)phosphoryl chloride, t-butoxy(methyl)thiophosphoryl chloride, t-butoxy(methoxy)phosphoryl chloride. Other phosphoryl chlorides may include t-butoxy(N-morpholino)phosphoryl chloride, t-butoxy(ethoxyethylamino)phosphoryl chloride, t-butoxy(methythioxy)phosphoryl chloride, and the like. Such phosphorylating agents are utilized to yield the corresponding phosphorothioate, phosphoramidate, phosphotriester, alkylphosphonates, and phosphodiester xylo nucleotides.

Even enzymatic phosphorylation is possible, as for example the phosphorylation of 9-(β-D-xylofuranosyl)guanine by nucleoside phosphotransferase from *Pseudomonas trifolii as* per the procedure of Suzaki, S., Yamazaki, A. Kamimura, A., Mitsugi, K., and Kumashior, I. (1970), *Chem*. *Pharm*. *Bull. (Tokyo)*, 18:172.

1-(β-D-Xylofuranosyl)uracil 5'-phosphate was identified but not separated from its 3' isomer as reported by Holy, A. and Sorm, F. (1969), *Coll*. *Czech*. *Chem*. *Commun.,* 34:1929. Also, 9-(2'-O-benzyl-β-D-xylofuranosyl)adenine 5'-phosphate was obtained as an intermediate by Hubert-Habart, M. and Goodman, L. (1969), *Chem. Commun*., 740. Removable of the benzyl blocking group would give the desired unblocked nucleotide.

Additionally, the alkylphosphonates can be prepared by the method of Holy, A. (1967), *Coll*. *Czech. Chem*. *Commun.,* 32:3713. Phosphorothioates have also been prepared by treatment of the corresponding nucleoside with trisimidazolyl-1-phosphinesulfide followed by acid hydrolysis with aqueous acetic acid, Eckstein, F. (1966 & 1970), *J. Am*. *Chem*. *Soc.,* 88:4292 & 92:4718, respectively. A more preferred method is by selective thiophosphorylation by thiophosphorylchloride in triethylphosphate, Murray, A.W. and Atkinson, M.R. (1968), *Biochemistry,* 7:4023.

The appropriate phosphorylated xylo nucleotide is then activated for nucleophilic displacement at its 3' position by reacting the 3'-hydroxyl group of the xylo compound with an appropriate anhydride, chloride, bromide, acyloxonium ion, or through an anhydro or cyclo nucleoside or the like to convert the 3'-hydroxyl group of the xylo nucleoside to an appropriate leaving group.

In a further method of synthesis, treatment of 2',3'-anhydroadenosine with sodium ethylmercaptide gives 9-[3-deoxy-3-(ethylthio)-β-D-xylofuranosyl]adenine. Treatment of this compound with a first synthon nucleophile may generate a terminal 2-ethylthio arabinofuranosyl nucleoside that could be desulfurized to yield the corresponding 2'-deoxynucleoside.

If during phosphorylation or conversion of the xylo 3'-hydroxyl to a 3'-activated leaving group stereospecific diastereomers are not obtained, after completion of the phosphorylation or conversion of the 3'-hydroxyl to an activated leaving group, the Rp and Sp diastereomers of these compounds will then be isolated by HPLC. This will yield pure diastereomers in a stereospecific form ready for use as the second synthons of Scheme 1.

Additional objects, advantages, and novel features of this invention will become apparent to those skilled in the art upon examination of the following examples thereof, which are not intended to be limiting.

### EXAMPLE 1

### Xyloadenosine

### Method A - Condensation Reaction

Adenine is condensed with 1,2,3,5-tetra-O-acetyl-D-xylopentofuranoside in the presence of TiCl₄ as the condensation catalyst in a polar solvent utilizing the method of Szarek, W.A., Ritchie, R.G.S., and Vyas, D.M. (1978), *Carbohydr*. *Res*., 62:89.

### Method B - Alkylation Reaction

8-Mercaptoadenine is alkylated with 5-deoxy-5-iodo-1,2-O-isopropylidine-xylofuranose followed by treatment with acetic acid/acetic anhydride/sulfuric acid and then ammonia. This yields an 8-5'-anhydro intermediate nucleoside that is oxidized with aqueous N-bromosuccinimide to give the sulfoxide. This is blocked with benzoic anhydride and after a Pummerer rearrangement can be desulfurized with Rainey nickel to give 9-β-D-xylofuranosyladenine as per the procedure of Mizuno, Y., Knaeko, C., Oikawa, Y., Ikeda, T, and Itoh, T. (1972), J. *Am. Chem*. *Soc*., 94:4737.

### EXAMPLE 2

### 3-Deaza-9-(β-D-xylofuranosyl)guanine

In a manner similar to Example 1, Method A, 3-deazaguanine is condensed with 1,2,3,5-tetra-O-acetyl-D-xylopentofuranoside to yield the title compound.

### EXAMPLE 3

### N⁶-Benzoyl-9-(2'-Deoxy-2'-fluoro-threo-pentofuranosyl)adenine

In a manner similar to Example 1, Method A, N⁵-benzoyladenine is condensed with 1,3,5-tri-O-acetyl-2-deoxy-2-fluoro-D-threo-pentofuranoside to yield the title compound.

### EXAMPLE 4

### 1-(2'-Deoxy-2'-methoxy-β-D-xylofuranosyl)uridine

In a manner similar to Example 1, Method A, uracil is condensed with 1,3,5-tri-O-acetyl-2-deoxy-2-methoxy-D-threo-pentofuranoside to yield the title compound.

### EXAMPLE 5

### 1-(2'-Deoxy-2'-O-allyl-β-D-threo-pentofuranosyl)cytosine

In a manner similar to Example 1, Method A, cytosine is condensed with 1,3,5-tri-O-acetyl-2-deoxy-2-O-allyl-D-threo-pentofuranoside to yield the title compound.

### EXAMPLE 6

### Xyloguanosine

### Method A

In a manner similar to Example 1, Method A, guanine is condensed with 1,2,3,5-tetra-O-acetyl-D-xylopentofuranoside to yield the title compound.

### Method B

The chloromercury derivative of 2-acetamido-6-chloropurine is condensed with 2,3,5-tri-O-acetyl-β-D ribofuranosylpurine utilizing the method of Lee et. al. (1971), *J. Med*. *Chem.,* 14:819. The condensation product was treated with ammonia to yield 2-amino-6-chloro-9-(β-D-xylofuranosyl)purine. Further treatment with sodium hydroxyethylmercaptide gives the title compound.

### EXAMPLE 7

### 2-Amino-6-mercapto-9-(β-D-xylofuranosyl)purine

2-Amino-6-chloro-9-(β-D-xylofuranosyl)purine as prepared by the Example 6, Method B, is treated with sodium hydrosulfide to give the title compound.

### EXAMPLE 8

### 9-(2'-Deoxy-2'-methyl-β-D-threo-pentofuranosyl)guanine

In a manner similar to Example 1, Method A, guanine is condensed with 1,3,5-tri-O-acetyl-2-deoxy-2-methyl-D-threo-pentofuranoside to yield the title compound.

### EXAMPLE 9

### 2-Amino-xyloadenosine

2-amino-8-mercaptoadenine is treated in the manner as per Example 6, Method B to yield the title compound.

### EXAMPLE 10

### Carbocyclic Xyloadenosine

5-Amino-4,6-dichloropyrimidine is treated with (±)-4α-amino-2α,3β-dihydroxy-1α-cyclopentanemethanol to give a pyrimidine intermediate that is aminated and ring closed to yield the carbocyclic analog of xylofuranosyladenine as per the procedure of Vince, R. and Daluge, S. (1972), *J. Med. Chem.,* 15:171.

### EXAMPLE 11

### Carbocyclic Xyloinosine

5-Amino-6-chloro-pyrimidyl-4-one when treated with (±)-4α-amino-2α,3β-dihydroxy-1α-cyclopentanemethanol will give a pyrimidine intermediate that is then aminated and ring closed to yield the carbocyclic analog of xylofuranosylinosine as per the procedure of Example 8.

### EXAMPLE 12

### O²,3'-Cyclothymidine

### Method A

3'-O-Mesylthymidine is treated with boiling water and the pH is adjusted to pH 4-5 according to the procedure of Miller, N. and Fox, J.J. (1964), *J. Org. Chem.,* 29:1771 to yield the title compound. This same compound can also prepared from 3'-deoxy-3'-iodothymidine by treatment with silver acetate in acetonitrile.

### Method B

O²,3'-Cyclothymidine and other 2'-deoxynucleosides are prepared by the treatment of the appropriate nucleoside with (2-chloro-1,1,3-trifluoroethyl)diethylamine in dimethylformamide according to the procedure of Kowollik, G., Gaertner, K., and Langen, P. (1969), *Tetrahedron Lett*., 3863.

### EXAMPLE 13

### O²,3'-Cyclouridine

3'-O-tosyluridine is treated with t-butoxide according to the procedure of Letters, R. and Michelson, A.M. (1961), *J*. *Chem. Soc.,* 1410. This compound is also prepared by treatment of 3'-O-mesyl-2',5'-di-O-trityluridine with sodium benzoate in dimethylformamide followed by detritlation with hydrochloric in chloroform.

### EXAMPLE 14

### S²,3'-Cyclo-2-thiothymidine

### Method A

3'-O-mesyl-O²,5'-cyclothymidine is subjected to methanolysis followed by sulfhydryl ion attack. The S²,3'-cyclo linkage is then opened up with base to yield 2',3'-dideoxy-3'-mercapto-1-(β-D-xylofuranosyl)thymidine as per the procedure of Wempen, I. and Fox, J.J. (1969), *J. Org*. *Chem*., 34:1020.

### Method B

S²,3'-Cyclo-2-thiouridine is also prepared from 2-thiouridine by the method of Doerr, I.L. and Fox, J.J. (1967), *J. Am. Chem*., 89:1760.

### EXAMPLE 15

### N⁶,5'-Cyclothymidine

5'-O-Trityl-3'-O-mesylthymidine is treated with sodium azide to yield N⁶,5'-cyclothymidine as one of the products. 5'-O-trityl-3'-O-mesylthymidine is also cyclizable to O²,3'-cyclothymidine.

### EXAMPLE 16

### 8,3'-Cycloadenosine

The anhydro ring from the 3' position of the sugar to the 8 position of the purine ring is formed by treatment of 5'-O-acetyl-8-bromo-2' (or 3')-O-p-toluenesulfonyladenosine with thiourea to yield the 8,3'-thiocyclonucleoside (as well as the corresponding 8,2') product as per the procedure of Ikehara, M. and Kaneko, M. (1970), *Tetrahedron,* 26:4251.

### EXAMPLE 17

### 8,3'-Cycloguanosine

The title compound is prepared as per Example 16 utilizing 8-bromoguanosine. Both this compound and the compound of Example 16 can be oxidized to their corresponding sulfoxides via tert-butyl hypochlorite in methanol or treated with chlorine in methanolic hydrogen chloride to yield the 3'-sulfo-8-chloro analog in a procedure analogous with that of Mizuno, Y., Kaneko, O., and Oikawa, Y. (1974), *J. Org. Chem.,* 39:1440.

### EXAMPLE 18

### 1-(3'-Bromo-3'-deoxy-N⁴,2',5'-O-triacetylxylofuranosyl) cytosine

The title compound is prepared by treating N⁴-acetylcytidine with acetyl bromide according to the procedure of Marumoto, R. and Honjo, M. (1974), *Chem*. *Pharm*. *Bull.,* 22:128.

### EXAMPLE 19

### 9-(3-Deoxy-3-fluoro-β-D-xylofuranosyl)adenine

Treatment of 9-(2,3-anhydro-5-O-benzoyl-β-D-ribofuranosyl)-N,N-dibenzoyl (or N-pivaloyl)adenine with tetraethylammonium fluoride in hot acetonitrile followed by deacylation with sodium methoxide yields 9-(3-deoxy-3-fluoro-β-D-xylofuranosyl)adenine as per the procedure of Lichtenthaler, F.W., Emig, P., and Bommer, D. (1969), *Chem*. *Ber.,* 102:964.

### EXAMPLE 20

### 9-(3-Deoxy-3-fluoro-β-D-xylofuranosyl)guanine

In a like manner to Example 18 the corresponding guanosine compound will be prepared from the corresponding 2,3-anhydro guanosine.

### EXAMPLE 21

### 9-(3'-Chloro-3'-deoxy-β-D-xylofuranosyl)hypoxanthine

5'-O-Acetylinosine is treated with triphenylphosphine and carbon tetrachloride to yield the title compound according to the procedure of Haga, K., Yoshikawa, M., and Kato, T. (1970), *Bull. Chem*. *Soc*. *Jpn.,* 43:3992.

### EXAMPLE 22

### 9-(2-O-Acetyl-3-chloro-3-deoxy-5-O-pivaloyl-β-D-xylofuranosyl)-6-pivalamidopurine

The title compound is prepared via an intermediate 2',3'-O-acyloxonium ion utilized to introduce a halogen atom at the 3' position and convert the ribo configuration of a nucleoside into the corresponding 3'-halo-3'-deoxy xylo nucleoside. The acyloxonium ion is generated *in situ* by treatment of 2',3'-O-methoxyethylidineadenosine with pivaloyl chloride in hot pyridine. Attack by chloride gives the title compound. Hypoxanthine and guanine nucleoside react in a similar manner. Sodium iodide will be used to generate the corresponding 3'-iodides according to the procedure of Robins, M.J., Fouron, Y., and Mengel, R. (1974), *J*. *Org*. *Chem.,* 39:1564.

### EXAMPLE 23

### 9-(2,5,-Di-O-acetyl-3-bromo-3-deoxy-β-D-xylofuranosyl)adenine

This compound is prepared by a *in situ* acyloxonium ion generated by treating 3',5'-di-O-acetyladenosine with boron trifluoride etherate followed by phosphorus tribromide according to the procedure of Kondo, K., Adachi, T., and Inoue, I. (1977), *J. Org*. *Chem.,* 42:3967. The title compound can also be formed by treating adenosine with tetraacetoxysilane and phosphorus tribromide in the presence of boron trifluoride etherate.

### EXAMPLE 24

### 1-(β-D-2'-Deoxy-2'-fluoro-threo-pentofuranosyl)uracil

In a manner similar to Example 3, uracil is condensed with 1,3,5,-tri-O-acetyl-2-deoxy-2-fluoro-D-threo-pentofuranoside to yield the title compound.

### EXAMPLE 25

### 1-(β-D-2'-Deoxy-2'-fluoro-threo-pentofuranosyl)guanine

In a manner similar to Example 3, guanine is condensed with 1,3,5,-tri-O-acetyl-2-deoxy-2-fluoro-D-threo-pentofuranoside to yield the title compound.

### EXAMPLE 26

### 1-(β-D-2'-Deoxy-2'-fluoro-threo-pentofuranosyl)cytosine

In a manner similar to Example 3, cytosine is condensed with 1,3,5,-tri-O-acetyl-2-deoxy-2-fluoro-D-threo-pentofuranoside to yield the title compound.

### EXAMPLE 27

### O²,3'-Cyclo-2'-deoxycytidine

The title compound is prepared by heating the 3'-O-sulfamate as per the procedure of Schuman, D., Robins, M.J., and Robins, R.K. (1970), *J. Am*. *Chem*. *Soc*., 92:3434.

### EXAMPLE 28

### Sp and Rp Xyloadenosine 5'-Monophosphate

N⁶-Benzoyl-xyloadenosine is phosphorylated with phosphoryl chloride in pyridine and acetonitrile at 0°C. The reaction will be quenched with ice water, rendered basic and added to an activated charcoal column. After elution with ethanol/water/concentrated ammonium hydroxide the solvent is evaporated to dryness and the residue dissolved in water and passed through an ion exchange column. The benzoyl blocking group is removed in concentrated ammonium hydroxide followed by separation of the diastereomers by HPLC to yield the title compound.

### EXAMPLE 29

### Sp and Rp 1-(β-D-2'-Deoxy-2'-fluoro-threo-pentofuranosyl)uracil 5'-t-butoxy(methyl)phosphonate

1-(β-D-2'-deoxy-2'-fluoro-threo-pentofuranosyl)thymine will be phosphorylated with t-butoxy(methyl)phosphoryl chloride in trimethylphosphate at 0°C for 3 hrs. The solution is added to cold anhydrous ether. The racemic precipitate is taken up in acetonitrile and the Sp and Rp diastereomers of the title compound separated by HPLC utilizing a gradient of acetonitrile and triethylammonium acetate buffer.

### EXAMPLE 30

### Sp and Rp 1-(β-D-2'-Deoxy-2'-fluoro-threo-pentofuranosyl)cytosine 5'-t-butoxy(methyl)phosphonate

1-(β-D-2'-deoxy-2'-fluoro-threo-pentofuranosyl)cytosine will be phosphorylated and purified as per the procedure of Example 29 to give the diastereomers of the title compound.

### EXAMPLE 31

### Sp and Rp N⁵-Benzoyl-9-(β-D-2'-Deoxy-2'-fluoro-threo-pentofuranosyl)adenine 5'-t-butoxy(methyl)phosphonate

N⁶-Benzoyl-9-(β-D-2'-deoxy-2'-fluoro-threo-pentofuranosyl)adenine will be phosphorylated and purified as per the procedure of Example 29 to give the diastereomers of the title compound.

### EXAMPLE 32

### Sp and Rp 9-(β-D-2'-Deoxy-2'-fluoro-threo-pentofuranosyl)guanine 5'-t-butoxy(methyl)phosphonate

9-(β-D-2'-Deoxy-2'-fluoro-threo-pentofuranosyl)guanine will be phosphorylated and purified as per the procedure of Example 29 to give the diastereomers of the title compound.

### EXAMPLE 33

### Sp and Rp Xylofuranosyluracil 5'-t-butoxyphosphorothioate

Xylofuranosyluracil will be phosphorothioated with t-butoxythiophosphorylchloride in triethylphosphate utilizing the method of Murray, A.W. and Atkinson, M.R. (1968), *Biochemistry,* 7:4023. The diastereomers of the title compound are separated by HPLC.

### EXAMPLE 34

### Sp and Rp 9-(2'-Deoxy-2'-methyl-β-D-threo-pentofuranosyl)guanine 5'-Methylphosphonate

9-(2'-Deoxy-2'-methyl-β-D-threo-pentofuranosyl)guanine will be alkylphosphonated utilizing the procedure of Holy, A. (1967), *Coll*. *Czech. Chem*. *Commun*., 32:3713. The racemic phosphorylation product is separated into its Sp and Rp diastereomers using HPLC chromatography to yield the title compound.

### EXAMPLE 35

### Sp and Rp 9-(2'-Deoxy-β-D-threo-pentofuranosyl)hypoxanthine 5'-Phosphormorpholidate

9-(2'-Deoxy-β-D-threo-pentofuranosyl)hypoxanthine is phosphorylated according to the procedure of Example 28. The resulting 5'-phosphate intermediate will be phosphormorpholidated by treatment with activated with dicyclohexylcarbodiimide in the presence of morpholine according to the procedure of Moffatt, J.G. and Khorana, H.G. (1961), *J. Am*. *Chem*. *Soc*., 83:3752 to yield the racemic title compound. The diastereomers of the product are separated by HPLC.

### EXAMPLE 36

### Sp and Rp 9-(2'-Deoxy-2'-O-allyl-β-D-threo-pentofuranosyl)cytosine 5'-Phosphate

9-(2'-Deoxy-2'-allyl-β-D-threo-pentofuranosyl)cytosine will be phosphorylated according to the procedure of Example 28 to yield the racemic title compound. The diastereomers are separated by HPLC.

### EXAMPLE 37

### Sp and Rp 9-(2'-Deoxy-2'-methoxy-β-D-threo-pentofuranosyl)-uracil 5'-Phosphate

9-(2'-Deoxy-2'-methoxy-β-D-threo-pentofuranosyl)uracil will be phosphorylated according to the procedure of Example 28 to yield the racemic title compound. The diastereomers are separated by HPLC.

### EXAMPLE 38

### Sp and Rp 3-Deaza-9-(xylofuranosyl)guanine 5'-Phosphate

3-Deaza-9-(xylofuranosyl)guanine will be phosphorylated according to the procedure of Example 28 to yield the racemic title compound. The diastereomers are separated by HPLC.

### EXAMPLE 39

### Sp and Rp Xyloguanosine 5'-Phosphorothioate

Xyloguanosine will be phosphorothioated with thiophosphoryl chloride according to the procedure of Example 28 to yield the racemic title compound. The diastereomers are separated by HPLC.

### EXAMPLE 40

### Sp and Rp Carbocyclic Xyloadenosine 5'-Phosphate

In a like manner to Example 28, carbocyclic xyloadenosine will be treated with phosphoryl chloride to yield the racemic title compound. The diastereomers will be separated by HPLC.

### EXAMPLE 41

### Activated 3'-Deoxy-3'-Active Leaving Group Phosphorylated Nucleosides

The 3'-halo nucleotides can be treated with methoxide to give an unstable 2',3'-anhydro intermediate that slowly forms the corresponding 3,3'-cyclonucleoside. The cyclonucleoside in turn can undergo nucleophilic attack to yield other 3'-deoxy-3'-substituted derivatives, as for instance, the tosyl, triflate, trichloroacetimidate or other active species.

### EXAMPLE 42

### N⁶-Benzoyl-9-(3'-Deoxy-3'-tosyl-2'-deoxy-2'-fluoro-β-D-threo-pentofuranosyl)adenine 5'-Rp t-Butoxy(methyl)phosphonate

N⁶-Benzoyl-9-(2'-deoxy-2'-fluoro-β-D-threo-pentofuranosyl)adenine 5'-Rp t-butoxy(methyl)phosphonate will be treated with p-toluenesulfonylchloride in pyridine as per the procedure of Reist, E.J., Bartuska, V.J., Calkins, D.F., and Goodman, L. (1965), *J. Org*. *Chem.,* 30:3401 to yield the title compound.

### EXAMPLE 43

### 9-(3'-Deoxy-3'-tosyl-2'-deoxy-2'-methoxy-β-D-threo-pentofuranosyl)uracil 5'-Rp t-Butoxy(methyl)phosphonate

9-(2'-Deoxy-2'-methoxy-β-D-threo-pentofuranosyl)uridine 5'-Rp t-butoxy(methyl)phosphonate will be treated with p-toluenesulfonylchloride in pyridine according to the procedure of Example 42 to yield the title compound.

### EXAMPLE 44

### 9-(3'-Deoxy-3'-tosyl-2'-deoxy-2'-fluoro-β-D-threo-pentofuranosyl)uracil 5'-Rp t-Butoxy(methyl)phosphonate

9-(2'-Deoxy-2'-fluoro-β-D-threo-pentofuranosyl)uridine 5'-Rp t-butoxy(methyl)phosphonate will be treated with p-toluenesulfonylchloride in pyridine according to the procedure of Example 42 to yield the title compound.

### EXAMPLE 45

### 9-(3'-Deoxy-3'-tosyl-2'-deoxy-2'-fluoro-β-D-threo-pentofuranosyl)cytosine 5'-Rp t-Butoxy(methyl)phosphonate

9-(2'-Deoxy-2'-fluoro-β-D-threo-pentofuranosyl)cytosine 5'-Rp t-butoxy(methyl)phosphonate will be treated with p-toluenesulfonylchloride in pyridine according to the procedure of Example 42 to yield the title compound.

### EXAMPLE 46

### 9-(3'-Deoxy-3'-tosyl-2'-deoxy-2'-fluoro-β-D-threo-pentofuranosyl)guanine 5'- Phosphate

9-(2'-Deoxy-2'-fluoro-β-D-threo-pentofuranosyl)guanine 5'-Sp phosphate will be treated with p-toluenesulfonyl chloride in pyridine according to the procedure of Example 42 to yield the title compound.

### EXAMPLE 47

### 9-(3'-Deoxy-3'-tosyl-2'-deoxy-2'-O-allyl-β-D-threo-pentofuranosyl)thymine 5'- Phosphate

9-(2'-deoxy-2'-O-allyl-β-D-threo-pentofuranosyl)thymine 5'-Rp phosphate will be treated with p-toluenesulfonyl chloride in pyridine according to the procedure of Example 42 to yield the title compound.

### EXAMPLE 48

### 3'-Deoxy-3'-trifluoromethanesulfonylxyloguanosine 5'- Phosphorothioate

Xyloguanosine 5'-Sp phosphorothioate will be treated with trifluoromethane sulfonic acid anhydride in the presence of a sodium hydride to yield the title compound.

### EXAMPLE 49

### Carbocyclic3'-Deoxy-3'-trifluoromethanesulfonylxyloadenosine 5'- Phosphate

In a like manner to Example 48, carbocyclic xyloadenosine 5'-Rp phosphate will be treated with trifluoromethane sulfonic acid to yield the title compound.

### EXAMPLE 50

### S²,3'-Cyclo-2-thiothymidine

S²,3'-Cyclo-2-thiothymidine is prepared from 3'-O-mesyl-O²,5'-cyclothymidine via methanolysis followed by sulfhydryl ion attack. The S²,3'-cyclo linkage is then opened up with base to yield 2',3'-dideoxy-3'-mercapto-1-(β-D-xylofuranosyl)thymidine, Wempen, I. and Fox, J.J. (1969), *J. Org*. *Chem.,* 34:1020. The 3' position will then be activated to nucleophilic attack via an active leaving group such as conversion of the mercapto to a tosyl leaving group. In a like manner S²,3'-Cyclo-2-thiouridine prepared from 2-thiouridine by the method of Doerr, I.L. and Fox, J.J. (1967), *J. Am. Chem.*, 89:1760, can be ring opened and then derivatized with an activated leaving group such as a tosylate.

### EXAMPLE 51

### Synthesis of 2'-Deoxy-2'-fluoro substituted CGA CTA TGC AAC TAC Rp Methylphosphonate Linked Oligonucleotide

1-(2'-Fluoro-2'-deoxy-β-D-ribofuranosyl)cytosine 5'-Rp methylphosphonate will be attached via its 3' hydroxyl to CPG beads in a standard manner as practiced in automated nucleic acid synthesis. This nucleotide forms the first synthon 1

### (a) Activation of Synthon 1

The beads are washed with acetonitrile and treated with 1.1 equivalents of sodium hydride in acetonitrile to form an anion on the methylphosphonate moiety.

### (b) Addition of Synthon 2 and Coupling of Synthons 1 and 2

2.0 Equivalents of N⁶-Benzoyl-9-(3'-deoxy-3'-tosyl-2'-deoxy-2'-fluoro-β-D-threo-pentofuranosyl)adenine 5'-Rp t-butoxy(methyl)phosphonate in acetonitrile is added with stirring. After completion of the nucleophilic reaction and formation of the cytosine-adenine dimer as judged by tlc, the beads are filtered and washed with acetonitrile.

### (c) Removal of t-Butoxy Blocking Group

The beads are re-suspended in acetonitrile and 1.5 equivalents of trichloracetic acid is added. The reaction is stirred to remove the t-butoxy blocking group on the terminal 5'-methylphosphonate group of the adenosine nucleotide, followed by washing with acetonitrile.

### (d) Cycling

The reaction is cycled to step (a), followed by addition of the next synthon 2 nucleotide at step (b), and deblocking at step (c). The reaction is further cycled for each nucleotide making up the specific sequence of the oligonucleotide. After the addition of the penultimate nucleotide its phosphate moiety is activated at step (a) and the final nucleosidic unit is added at step (b) as a xylofuranosyl nucleoside. The oligonucleotide is concurrently deblocked and removed from the CPG beads by treatment with concentrate ammonium hydroxide.

### EXAMPLE 52

### Isolation of All-Sp or All Rp 5'-O-(1-thiotriphosphate) Nucleoside

5'-O-(1-thiotriphosphate) deoxynucleosides and ribonucleosides are isolated using C-18 reverse phase high performance liquid chromatography (HPLC) using columns packed with ODS Hypersil (Shandon Southern, Runcon, UK) and eluted with an isocratic mixture of solvent A (30 mM potassium phosphate containing 5 mM tetrabutylammonium ion, pH 7.0) and solvent B (5 mM tetrabutylammonuium hydroxide in methanol). Alternatively, effective separation is achieved using 100 mM triethylammonium bicarbonate, pH 7.5, containing a linear gradient of acetonitrile from 0% to 15% over 20 minutes.

To establish the purity of such HPLC separated enantiomers the HPLC separated Sp and Rp deoxynucleotide enantiomers are compared to commercially available deoxynucleoside 5'-O-(1-thiotriphosphates) available from E.I. Dupont, Wilmington, DE.

### EXAMPLE 53

### Synthesis of Phosphorothioate Extension having Substantially All-Rp Intersugar Linkages of a Racemic Phosphorothioate Oligonucleotide

Enzymatic synthesis of an all-Rp phosphorothioate extension of a racemic phosphorothioate oligonucleotide primer is effected using the modified T7 DNA polymerase I, Sequenase-™ (U.S. Biochemicals Corp, Cleveland, OH). This T7 DNA polymerase is used to extend an 18 mer phosphorothioate oligonucleotide primer hybridized to a 21 mer natural phosphodiester oligonucleotide. 30 picomoles (pmol) of primer and template in a 1X Sequenase™ reaction buffer (U.S. Biochemicals Corp., Cleveland, OH) (final vol 10 µl) are heated for 5 minutes at 95 °C and slowly cooled to room temperature. 180 pmol of deoxy 5'-[alpha-³⁵S]cytidine triphosphate and Sequenase™ enzyme (U.S. Biochemicals Corp., Cleveland, OH) are added and incubated at 37 °C for 20 minutes. The product is analyzed via polyacrylamide gel electrophoresis (PAGE) using a 20% polyacrylamide/7M urea denaturing gel. The autoradiograph of the product is compared to a control reaction absent primer/template. The final product is subjected to further characterization by, for example, enzymatic degradation. One such degradation is snake venom phosphatase degradation. A snake venom phosphatase degradation of dinucleoside monophosphorothioate synthesized using *E*. *coli* DNA polymerase I shows the dinucleoside to be of the Rp configuration.

### EXAMPLE 54

### Synthesis of Phosphorothioate CGA CTA TGC AAG TAC (SEQ ID NO:9) Oligonucleotide Having Substantially Pure Rp Intersugar Linkages

A large scale enzymatic synthesis of sequence specific all-Rp phosphorothioate oligonucleotide was effected utilizing a 55 mer natural phosphodiester template and a 41 mer natural phosphodiester primer. The template sequence was: GTA CTT GCA TAG TCG ATC GGA AAA TAG GGT TCT CAT CTC CCG GGA TTT GGT TGA G (SEQ ID NO: 7). The primer sequence was: CTC AAC CAA ATC CCG GGA GAT GAG AAC CCT ATT TTC CGA TC (SEQ ID NO:8). The template was selected to have a sequence complementary to a desired specific CGA CTA TGC AAG TAC (SEQ ID NO:9) sequence. A Sequenase™ buffer (U.S. Biochemicals Corp., Cleveland, OH) diluted from 5X down to 1X was used. The template and primer, both at concentrations of 20nM are added to 40 µL of this buffer. The template and primer were hybridized at 95 °c for 5 minutes and cooled to room temperature. After cooling the buffer was adjusted to 7 mM DTT. 20 µL 1:8 diluted Sequenase™ enzyme and 320 µM each of Sp GTPαS, CTPαS, ATPαS and TTPαS are then added. The reaction solution was adjusted to 140 µL with H₂O. It was incubated at 37 °C for 18 hours. The reaction solution was extracted 2X with a like volume of phenol in a standard manner and precipated in a standard manner by treatment with 2.5 volumes of 100% ethanol at -20°C, peltized, washed with 500µl 70% ethanol, peltized again and dried. The precipitate was suspended in 20 µL H₂O for 30 minutes then adjusted to 1 mM CaCl₂, 25 mM Tris HCl pH 8.0 in 40 µL H₂O. The solution was held at 95 °C for 5 minutes and snap cooled, i.e. very quickly cooled with ice. The template and primer were removed from the synthesized oligonucleotide by the addition of 4.6 µM DNase I and incubation at 37 °C for 10 minutes. The reaction mixture was phenol extracted 2X with precipated and precipitated with ethanol as above. The precipate was resuspended in H₂O and purfied using 20% polyacrylamide/7M urea gel electrophoresis coupled with SepPak™ chromatography (Millipore, Milford, MA).

In an alternate synthesis, Pst 1 restriction nuclease (Life Technologies, Inc., Gaithersburg, MD) was used to cleave the primer-bound phosphorothioate oligonucleotide at the restriction site. The desired CGA CTA TGC AAG TAC phosphorothioate oligonucleotide was purified using polyacrylamide/7M urea gel electrophoresis coupled with SepPak™ chromatography (Millipore, Milford, MA). Yields were optimized using enzymatic cascade effected by repetitive template-primer addition throughout the reaction. The cascade augmented synthesis yielded 75 A₂₆₀ units of the CGA CTA TGC AAG TAC all-Rp configuration phosphorothioate oligonucleotide from a 20 ml reaction.

### EXAMPLE 55

### Synthesis of Phosphorothioate Oligonucleotides Having a Racemic Mixture of Intersugar Linkages Using Automated DNA Synthesis.

Oligonucleotides are synthesized on an automated DNA synthesizer (Applied Biosystems model 380B) using hydrogenphosphonate chemistry in a standard manner. See Agrawal, S., Goodchild, J., Civeria, M.P., Thornton, A.H., Sarin, P.S., and Zamecnik, P.C. (1988) *Proc*. *Natl*. *Acad*. *Sci. USA*, 85:7079-7083. After the final coupling step, the phosphorothioate linkages are generated by oxidizing the bound oligomer with sulfur in carbon disulfide/triethylamine/pyridine. After sulfur oxidation, standard deblocking procedures with ammonium hydroxide are used to release the oligonucleotides from the support and remove base blocking groups. The phosphorothioate oligonucleotides are purified by oligonucleotide purification column (OPC; ABI, Foster City, CA) chromatography and HPLC, using a Beckman System Gold HPLC. The HPLC-purified oligonucleotides are then precipitated with ethanol and assessed for final purity by gel electrophoresis on 20% acrylamide/7 M urea or by analytical HPLC. The authenticity of the oligonucleotide sequence was assessed by oxidation with iodine in pyridine/water and standard sequencing methods. These oligonucleotides contain a mixture of all possible combinations of Rp and Sp isomers at each phosphorous linkage.

### EXAMPLE 56

### synthesis of Complementary DNA or RNA Sequences Using T7 RNA Polymerase for Thermodynamic and Kinetic Hybridization Analysis.

The synthesis of short complementary DNA oligonucleotides of natural phosphodiester linkages was performed utilizing standard automated synthesis on an ABI model 380B DNA Synthesizer. The oligonucleotides of correct length were purified by HPLC and sequenced by standard techniques.

T7 RNA polymerase was use for the synthesis of short, complementary RNA oligonucleotides for hybridization analysis. A large amount of T7 RNA polymerase at high concentrations was needed for the many cycles of initiation required to synthesize short RNAs. Due to this requirement, the T7 RNA polymerase was derived from a strain of *E. coli* that contained a T7 RNA polymerase expression vector, BL21/pAR1219, obtained from Brookhaven National Laboratory (Upton, NY). The isolation yielded approximately 300,000 to 500,000 units of T7 RNA polymerase from 2 l of cells, absorbance value =1.2 A₆₀₀. This was sufficiently concentrated for synthesis of short (10-30 nucleotide) RNA species. For synthesis, a T7 promoter and a template containing the complementary target sequence and T7 promoter hybridization sequence were synthesized using the ABI synthesizer (ABI, Foster City, CA). Template and promoter were purified by HPLC to ensure that the correct species was present for enzymatic synthesis. Synthesized products were purified on a 20% polyacrylamide/8M urea gel and sequenced by standard procedures.

### EXAMPLE 57

### Thermal Denaturation

Oligonucleotides (either phosphorothioate oligonucleotides of the invention or otherwise) were incubated with either the complementary DNA or RNA oligonucleotides at a standard concentration of 4 µM for each oligonucleotide in 100 mM ionic strength buffer (89.8 mM NaCl, 10 mM Naphosphate, pH 7.0, 0.2 mM EDTA). Samples were heated to 90 °C and the initial absorbance taken using a Guilford Response II spectrophotometer (Corning). Samples were then slowly cooled to 15°C and the change in absorbance at 260 nm monitored during the heat denaturation procedure. The temperature was elevated 1 degree/absorbance reading and the denaturation profile analyzed by taking the first derivative of the melting curve. Data was also analyzed using a two-state linear regression analysis to determine the Tₘ and delta G. The results of these tests are shown in Table 1.

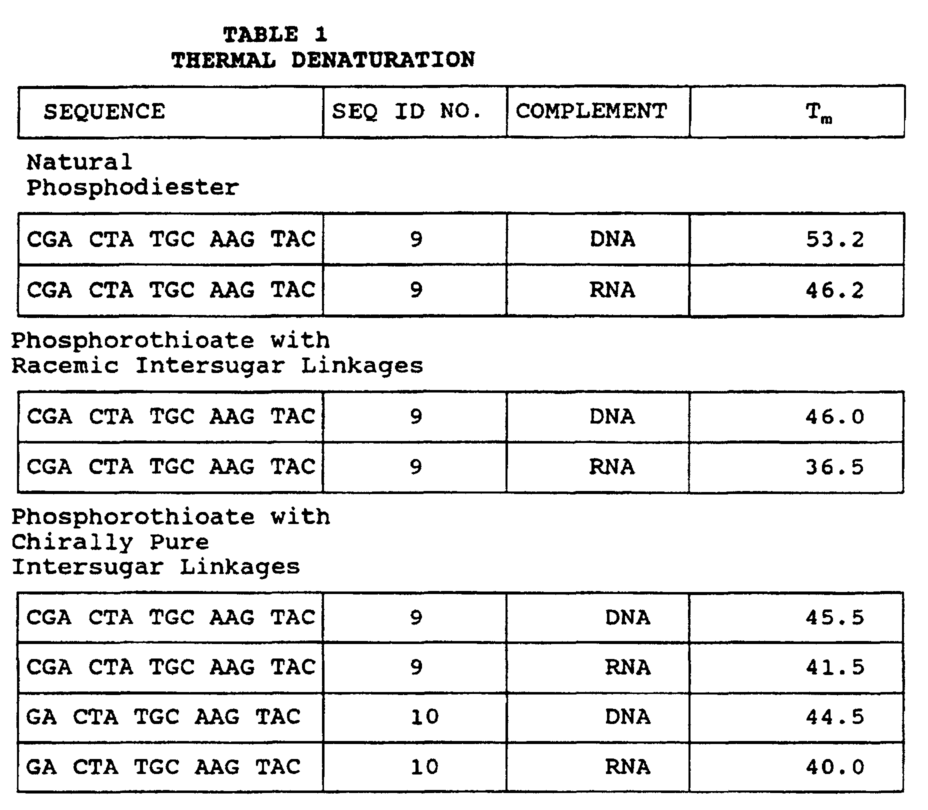

### EXAMPLE 58

### Synthesis of Radiolabeled Oligonucleotides

Filter binding assays are utilized to quantitate the binding stringencies of various phosphorothioate oligonucleotides; that is, their tendencies to hybridize and form heteroduplexes with DNA or RNA. These assays require radiolabeled oligonucleotides.

Phosphorothioate oligonucleotides having all-Rp intersugar linkages are synthesized by enzymatic methods from [³⁵S]-monomers that have been purified from Sp monomers. For automated synthesis of phosphorothioate oligonucleotides containing mixed chirality intersugar linkages, oligonucleotides are synthesized containing hydrogen phosphonates and then sulfurized in the presence of elemental [³⁵S] in a pyridine/carbon disulfide mixture. The resulting radiolabeled phosphorothioate oligonucleotide can be purified by OPC chromatography and HPLC. Target mRNA are applied to nitrocellulose filters and baked at 80°C for 2 hours, blocked and then hybridized with the radiolabeled phosphorothioate oligonucleotide. Binding stringency is assessed by quantitating radiolabeled oligonucleotide eluted from the filters after increases in temperature or increases in the ionic strength of an eluting buffer, as for instance, Tris NaCl buffer. Eluted oligonucleotides are also assessed for their mobility in an anion exchange HPLC protocol isocratically utilizing phosphate buffer. Results are compared to the mobility of standard oligonucleotides prepared having racemic mixtures of intersugar linkages.

### EXAMPLE 59

### Nuclease Digestion

Determination of the rate of nuclease degradation of the phosphorothioate oligonucleotides in media containing 10% fetal calf serum (FCS) was carried out in Dulbecco's Modified Essential Medium (DMEM) containing 10% heat inactivated FCS. Heat inactivation of the FCS was carried out at 55 °C for 1 hour prior to addition to media. Oligonucleotides having racemic and chirally pure intersugar linkages were separately tested for resistance to nuclease digestion. 66µg/ml of each oligonucleotide were separately added to medium and incubated at 37 °C, at the time intervals indicated in Table 2. 15 µl aliquots were removed and added to 15 µl of 9 M urea in 0.1 M Tris-HCl (pH 8.3), 0.1 M boric acid and 2 mM EDTA. Aliquots were mixed by vortex and stored at -20°C. Polyacrylamide gel electrophoresis (PAGE) analysis was on 20% polyacrylamide/7 M urea slab gels. Following electrophoresis, gels were stained using "Stains All" (Sigma Chem. Co., St. Louis, MO). Following de-staining, gels were analyzed via laser densitometry using an UltraScan XL device (Pharmacia LKB Biotechnology, Uppsala, Sweden). Integrations were performed and the data presented as the percentage decrease from full length (n) prior to incubation to n-1. These results are shown in Table 2 for the oligonucleotide sequence CGA CTA TGC AAG TAC (SEQ ID NO: 9) having Rp-chirally pure intersugar linkages.

**TABLE 2**

| **NUCLEASE DIGESTION** Incubation in 10% Fetal Calf Serum Digestion of Oligonucleotide of Length n to Length n-1 | | |
|---|---|---|
| Time(Hours) | Phosphorotioate with Racemic Intersugar Linkages | Phosphorothioate with Chirally Pure Intersugar Linkages |
| 0 | 0 | 0 |
| 1 | 44 | 10 |
| 2 | 45 | 10 |
| 4 | 54 | 12 |
| 24 | 70 | 44 |
| 48 | 70 | 62 |

As is evident from Table 2, the phosphorothioate oligonucleotide having substantially chirally pure intersugar linkages showed greater resistance to nuclease degradation than did the phosphorothioate oligonucleotide having racemic intersugar linkages.

### EXAMPLE 60

### RNase H Analysis

Phosphorothioate oligonucleotides having racemic and substantially chirally pure intersugar linkages were analyzed for suseptibility to RNase H. oligonucleotides (2-fold molar excess to RNA) and 5 µg (3.1 kb) *in vitro* synthesized mRNA (using T7 RNA polymerase promoter) were incubated in 5 µl RNase H hybridization buffer for 30 minutes at 60 °C. Samples were slowly cooled to room temperature and then adjusted to 3.7 mg/ml BSA, 20 units *E. coli* RNase H (Promega), 142 mM DTT, 150 mM KCl, and 3 mM MgCl₂. Samples were incubated for 30 minutes at 37°C. Samples were then phenol extracted, ethanol precipitated, and analyzed by electrophoresis on 1.2% agarose gels following ethidium bromide staining. Markers were run on gels concurrently with the samples to determine approximate length of RNA samples.

### EXAMPLE 61

A patient suffering from psoriasis is treated with 10µg/kg body weight of oligonucleotide sythesized according to the method of Example 3, incorporated in a cream. Daily application of the cream continues until the condition is relieved.

### EXAMPLE 62

A patient infected with human papillomavirus HPV-11 is treated with oligonucleotide synthesized according to Example 3, having the sequence TTG CTT CCA TCT TCC TCG TC (SEQ ID NO: 4). 1000 µg/kg body weight of oligonucleotide is incorporated into a pharmaceutically acceptable carrier and administered by a single intravascular injection, repeated as necessary until the infection is resolved.

### EXAMPLE 63

A patient infected with *Candida Albicans* is treated with oligonucleotide synthesized according to Example 3, having the sequence TGT CGA TAA TAT TAC CA (SEQ ID NO:3). 100 µg/kg body weight doses of oligonucleotide are administered orally in a pharmaceutically acceptable carrier every six hours for one week or until the infection is abated.

## Claims

1. A sequence specific oligonucleotide having at least 10 units linked together and complementary to at least a portion of a sequence of a targeted RNA or DNA, wherein at least three of said nucleoside units are linked together by either substantially all Rp or all Sp phosphorothioate, alkylphosphonate, phosphotriester, or phosphoramidate, with the proviso that if said oligonucleotide is linked together by all phosphorothioate linkages then said oligonucleotide has less than all of said nucleoside units linked together by either all Rp or all Sp linkages.

2. The oligonucleotide of claim 1 wherein said phosphate linkages are selected from the group consisting of chiral Sp phosphorothioate, chiral Rp phosphorothioate, chiral Sp alkylphosphonate, chiral Rp alkylphosphonate, chiral Sp phosphoamidate, chiral Rp phosphoamidate, chiral Sp chiral phosphotriester or chiral Rp phosphotriester.

3. An oligonucleotide comprising at least 10 nucleoside units linked together via phosphate linkages, wherein:
at least one of the nucleoside units is a non-naturally occurring nucleoside unit having at the 2' position a substituent selected from the group consisting of OH, C₁₋₁₂ alkyl, C₁₋₁₂ substituted alkyl, F, Cl, Br, CN, CF₃, OCF₃, OCN, O-alkyl, substituted O-alkyl, S-alkyl, substituted S-alkyl, SOMe, SO₂Me, ONO₂, NO₂, N₃, NH₂, NH-alkyl, substituted NH-alkyl, OCH₂CH=CH₂, OCH=CH₂, OCH₂CCH, OCCH, aralkyl, heteroaralkyl, heterocycloalkyl, poly-alkylamino, substituted silyl, an RNA cleaving moiety, a group which improves the pharmacodynamic properties of an oligonucleotide, or a group which improves the pharmacokinetic properties of an oligonucleotide; and
at least two of the units are linked via chiral phosphate linkages.

4. The oligonucleotide of claim 3 wherein said chiral phosphate linkages are selected from the group consisting of chiral Sp phosphorothioate, chiral Rp phosphorothioate, chiral Sp alkylphosphonate, chiral Rp alkylphosphonate, chiral Sp phosphoamidate, chiral Rp phosphoamidate, chiral Sp chiral phosphotriester or chiral Rp phosphotriester.

5. The oligonucleotide of claim 3 wherein each of the phosphate linkages is a chiral phosphate linkage.

6. An oligonucleotide according to any one of claims 1 to 5 comprising a plurality of nucleoside units linked together by all Sp phosphotriester linkages or by all Rp phosphotriester linkages.

7. An oligonucloetide according to any one of claims 1 to 5 comprising a plurality of nucleoside units linked together by all Sp phosphoramidate linkages or by all Rp phosphoramidate linkages.

8. An oligonucleotide according to any one of claims 1 to 5 comprising at least 10 nucleoside units linked together by all Sp alkylphosphonate linkages or by all Rp alkylphosphonate linkages.

9. The oligonucleotide of claim 28 wherein said alkylphosphonate linkages are methylphosphonate linkages.

10. An oligonucleotide according to any one of claims 1 to 9 for use in medicine.

11. A method for synthesising the oligonucleotide of claim 1 comprising the steps of:
(a) selecting a first synthon having structure: 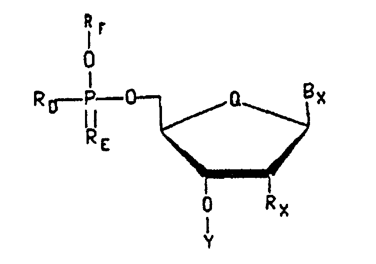
(b) selecting a second synthon having structure: 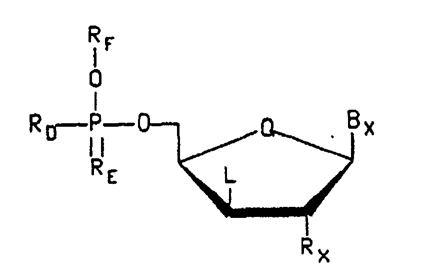
(c) contacting the second synthon with the first synthon in the presence of a base to effect nucleophilic attack of a 5'-phosphate of the first synthon at a 3'-position of the second synthon to yield a new first synthon via an inversion of configuration at the 3' position of the second synthon; and
(d) treating the new first synthon with a reagent to remove the blocking group R_{F};
wherein:
Q is O or CH₂;
R_{D} is methyl, O-alkyl, S-alkyl, amino or substituted amino;
R_{E} is O or S;
R_{F} is H or a labile blocking group;
R_{X} is H, OH, or a sugar derivatizing group;
B_{X} is a naturally occurring or synthetic nucleoside base or blocked nucleoside base;
L is a leaving group or together L and Bx are a 2-3' or 6-3' pyrimidine or 8-3' purine cyclo-nucleoside; and
Y is a stable blocking group, a solid state support, a nucleotide on a solid state support, or an oligonucleotide on a solid state support.

12. The method of claim 11 wherein L is selected from the group consisting of halogen, alkylsulfonyl, substituted alkylsulfonyl, arylsulfonyl, substituted arylsulfonyl, hetercyclcosulfonyl or trichloroacetimidate.

13. The method of claim 11 wherein L is selected from the group consisting of chloro, fluoro, bromo, iodo, p-(2,4-dinitroanilino)benzenesulfonyl, benzenesulfonyl, methylsulfonyl (mesylate), p-methylbenzenesulfonyl (tosylate), p-bromobenzenesulfonyl, trifluoromethylsulfonyl (triflate), trichloroacetimidate, acyloxy, 2,2,2-trifluoroethanesulfonyl, imidazolesulfonyl and 2,4,6-trichlorophenyl.

14. The method of claim 11 wherein said base is selected from the group consisting of sodium hydride, methylmagnesium chloride, t-butylmagnesium chloride or methyl-magnesium fluoride.

15. The method of claim 11 wherein R_{F} is an acid labile blocking group.

16. The method of claim 11 wherein Y is a hydrogenolysis sensitive blocking group.

17. The method of claim 11 wherein R_{F} is selected from the 5 group consisting of t-butyl, dimethoxytrityl or tetrahydropyranyl.

18. The method of claim 11 wherein the new first synthon has the structure:

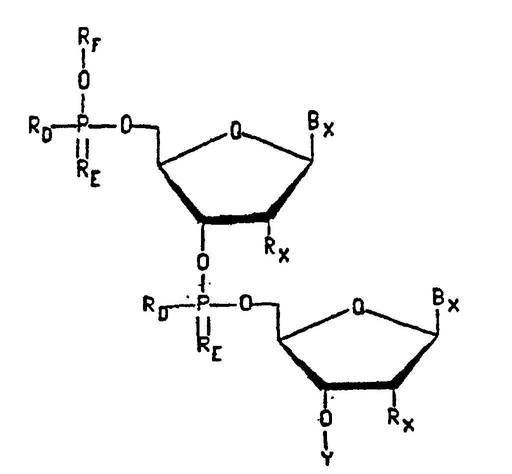

19. The method of claim 11 further comprising contacting said first synthon with said base in a solvent selected from the group consisting of acetonitrile, tetrahydrofuran and dioxane.

20. The method of claim 11 wherein said reagent used to remove blocking group R_{F} is an acid selected from the group consisting of trichloroacetic acid, acetic acid or trifluoromethane sulfonic acid.

21. A compound having the structure: 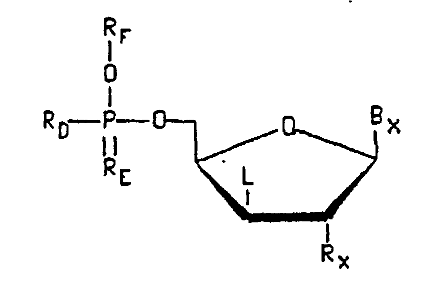 wherein:
Q is O or CH₂;
R_{D} is O^{⊖}, S^{⊖}, methyl, O-alkyl, S-alkyl, amino or substituted amino;
R_{E} is O or S;
R_{F} is H or a blocking group;
R_{X} is H, OH, or a sugar derivatizing group;
B_{X} is a nucleoside base, or a blocked nucleoside base; and
L is a leaving group or together L and BX are a 2-3' or 6'3' pyrimidine or 8-3' purine cyclo-nucleoside.

22. The compound of claim 21 wherein L is selected from the group consisting of halogen, alkylsulfonyl, substituted alkylsulfonyl, arylsulfonyl, substituted arylsulfonyl, hetercyclosulfonyl or trichloroacetimidate.

23. The compound of claim 21 wherein L is selected from the group consisting of chloro, fluoro, bromo, iodo, p-(2,4-dinitroanilino)benzenesulfonyl, benzenesulfonyl, methylsulfonyl (mesylate), p-methylbenzenesulfonyl (toxylate), p-bromobenzenesulfonyl, trifluoromethylsulfonyl (triflate), trichloroacetimidate, acyloxy, 2,2,2-trifluoroethanesulfonyl, imidazolesulfonyl and 2,4,6-trichlorophenyl.

24. The compound of claim 21 wherein R_{X} is H, OH, alkyl, alkenyl, alkynyl, substituted alkyl, alkenyl, alkynyl, F, Cl, Br, Cn, CF₃, OCF₃, OCN, O-alkyl, O-alkenyl, O-alkynyl, substituted O-alkyl, substituted O-alkenyl, substituted O-alkynl, S-alkyl, S-alkenyl, S-alkynyl, substituted S-alkyl, substituted S-alkenyl, substituted S-alkynyl, SOMe, SO₂Me, ONO₂, NO₂, N₃, NH₂, NH-alkyl, NH-alkenyl, NH-alkynyl, substituted NH-alkyl, substituted NH-alkenyl, substituted NH-alkynyl, OCH₂CH=CH₂, OCH=CH₂, OCH₂CCH, OCCH, aralkyl, aralkenyl, aralkynyl, heteroaralkyl, heteroaralkenyl, heteroaralkynyl, heterocycloalkyl, poly(alkylamino), substituted silyl, an RNA cleaving moiety, a group for improving the pharmacodynamic properties of an oligonucleotide or a group for improving the pharmacokinetic properties of an oligonucleotide.

25. The compound of claim 21 wherein R_{F} is selected from the group consisting of H, t-butyl, dimethoxytrityl or tetrahydrophyranyl.

26. The compound of claim 21 wherein Q is O.

27. The compound of claim 21 having Sp stereochemistry.

28. The compound of claim 21 having Rp stereochemistry.

29. An enzymatic method for synthesising the oligonucleotide of claim 2 said oligonucleotide having at least 10 nucleoside units joined together by either substantially all Sp or substantially all Rp phosphorus intersugar linkages comprising the steps of:
combining a sequence primer and a template;
adding an excess of all four nucleoside 5'-O-triphosphates having a desired 5'phosphate moiety; said triphosphate being one of essentially all Rp or all Sp triphosphates;
adding polymerase effective for extending the primer to form oligonucleotide complementary to said template; and
disassociating the resulting oligonucleotide from said primer and from the template.

30. The method of claim 29 wherein at least one step is repeated a plurality of times sufficient to promote the yield of said sequence specific phosphorothioate oligonucleotide.

31. An enzymatic method for synthesizing the oligonucleotide of claim 2 said oligonucleotide having at least 10 nucleoside units joined together by either substantially all Sp phosphorus intersugar linkages or substantially all Rp phosphorus intersugar linkages comprising the steps of:
combining a sequence primer and a template;
adding an excess of a racemic mixture of all four nucleoside 5'-O-(triphosphates) having a desired phosphate moiety;
adding a selected metal ion effective to promote preferential incorporation of either Rp or Sp triphosphates;
adding polymerase effective for extending the primer to form oligonucleotide complementary to said template; and
disassociating said the resulting oligonucleotide from said primer and from the template.

32. The method of claim 31 wherein said metal ion is magnesium.

33. The method of claim 31 wherein said metal ion is manganese.

34. The method of claim 29 or claim 31 further comprising the steps of:
prehybridizing said template and said primer by heating said template and said primer together; and
cooling said template and primer mixture prior to the addition of said triphosphates.

35. The method of claim 29 or claim 31 wherein said polymerase is T7 DNA polymerase, modified T7 DNA polymerase I, T7 RNA polymerase, T4 bacteriophage polymerase, modified T4 DNA polymerase, *M*. *luteus* polymerase, DNA poly Klenow fragment polymerase, *E. coli* RNA polymerase or *E*. *coli* DNA polymerase.

36. The method of claim 29 or claim 31 wherein disassociation from the primer is accomplished by means of Pst 1 restriction endonuclease, BamH1 restriction endonuclease, Smal restriction endonuclease or HinD III restriction endonuclease.

37. The method of claim 29 or claim 31 wherein the disassociation is achieved by DNAse I digestion.

## Patentansprüche

1. Sequenzspezifisches Oligonukleotid, das mindestes 10 Einheiten aufweist, die miteinander verbunden und komplementär zu mindestens einem Teil einer Sequenz einer Target-RNA oder -DNA sind, wobei mindestens drei der Nukleosid-Einheiten durch entweder im wesentlichen ausschließlich Rp- oder im wesentlichen ausschließlich Sp-Phosphorthioat, -Alkylphosphonat, -Phosphotriester oder -Phosporamidat miteinander verbunden sind, mit der Maßgabe, daß, wenn das Oligonukleotid durch ausschließlich Phosphoramidat-Verknüpfungen miteinander verbunden ist, dann die Nukleosid-Einheiten des Oligonukleotids nicht alle miteinander durch entweder ausschließlich Rpoder ausschließlich Sp-Verknüpfungen verbunden sind.

2. Oligonukleotid nach Anspruch 1, wobei die Phosphat-Verknüpfungen aus der Gruppe, bestehend aus chiralem Sp-Phosphorthioat, chiralem Rp-Phosphorthioat, chiralem Sp-Alkylphosphonat, chiralem Rp-Alkylphosphonat, chiralem Sp-Phosphoamidat, chiralem Rp-Phosphoamidat, chiralem Sp-Phosphotriester und chiralem Rp-Phosphotriester, ausgewählt sind.

3. Oligonukleotid, umfassend mindestens 10 Nukleosid-Einheiten, die miteinander durch Phosphat-Verknüpfungen verbunden sind, wobei: mindestens eine der Nukleosid-Einheiten eine nicht-natürlich vorkommende Nukleosid-Einheit ist, die an der 2'-Position einen Substituenten aufweist, der aus der Gruppe, bestehend aus OH, C₁₋₁₂ Alkyl, substituiertem C₁₋₁₂ Alkyl, F, Cl, Br, CN, CF₃, OCF₃, OCN, O-Alkyl, substituiertem O-Alkyl, S-Alkyl, substituiertem S-Alkyl, SOMe, SO₂Me, ONO₂, NO₂, N₃, NH₂, NH-Alkyl, substituiertem NH-Alkyl, OCH₂CH=CH₂, OCH=CH₂, OCH₂CCH, OCCH, Aralkyl, Heteroaralkyl, Heterocycloalkyl, Poly-Alkylamino, substituiertem Silyl, einem RNA-spaltenden Rest, einer Gruppe, welche die pharmakodynamischen Eigenschaften eines Oligonukleotids verbessert, oder einer Gruppe, welche die pharmakokinetischen Eigenschaften eines Oligonukleotids verbessert, ausgewählt ist; und
mindestens zwei der Einheiten durch chirale Phosphat-Verknüpfungen verbunden sind.

4. Oligonukleotid nach Anspruch 3, worin die chiralen Phosphat-Verknüpfungen aus der Gruppe, bestehend aus chiralem Sp-Phosphorthioat, chiralem Rp-Phosphorthioat, chiralem Sp-Alkylphosphonat, chiralem Rp-Alkylphosphonat, chiralem Sp-Phosphoamidat, chiralem Rp-Phosphoamidat, chiralem Sp-Phosphotriester und chiralem Rp-Phosphotriester, ausgewählt sind.

5. Oligonukleotid nach Anspruch 3, worin jede der Phosphat-Verknüpfungen eine chirale Phospat-Verknüpfung ist.

6. Oligonukleotid gemäß irgendeinem der Ansprüche 1 bis 5, umfassend eine Vielzahl von Nukleosid-Einheiten, die miteinander durch ausschließlich Sp-Phosphotriester-Verknüpfungen oder ausschließlich Rp-Phosphotriester-Verknüpfungen verbunden sind.

7. Oligonukleotid gemäß irgendeinem der Ansprüche 1 bis 5, umfassend eine Vielzahl von Nukleosid-Einheiten, die miteinander durch ausschließlich Sp-Phosphoramidat-Verknüpfungen oder ausschließlich Rp-Phosphoramidat-Verknüpfungen verbunden sind.

8. Oligonukleotid gemäß irgendeinem der Ansprüche 1 bis 5, umfassend mindestens 10 Nukleosid-Einheiten, die miteinander durch ausschließlich Sp-Alkylphosphonat-Verknüpfungen oder ausschließlich Rp-Alkylphosphonat-Verknüpfungen verbunden sind.

9. Oligonukleotid nach Anspruch 8, wobei die Alkylphosphonat-Verknüpfungen Methylphosphonat-Verknüpfungen sind.

10. Oligonukleotid gemäß irgendeinem der Ansprüche 1 bis 9 für die Verwendung in der Medizin.

11. Verfahren zum Synthetisieren des Oligonukleotids nach Anspruch 1, umfassend die Schritte:
(a) Auswählen eines ersten Synthons mit der Struktur: 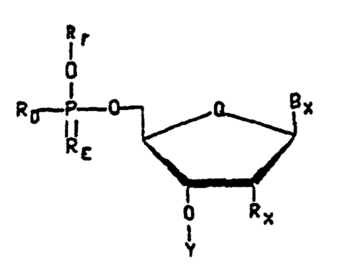
(b) Auswählen eines zweiten Synthons mit der Struktur: 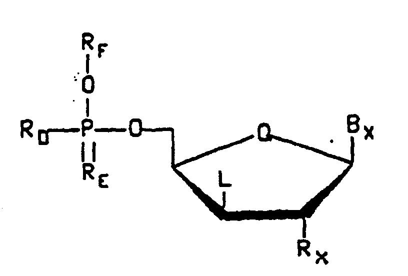
(c) Inkontaktbringen des zweiten Synthons mit dem ersten Synthon in der Gegenwart einer Base, um einen nukleophilen Angriff eines 5'-Phosphats des ersten Synthons an einer 3'-Position des zweiten Synthons zu bewirken, unter Erhalten eines neuen ersten Synthons über eine Inversion der Konfiguration an der 3'-Position des zweiten Synthons; und
(d) Behandeln des neuen ersten Synthons mit einem Reagens zur Entfemung der blockierenden Gruppe R_{F};
wobei:
Q O oder CH₂ ist;
R_{D} Methyl, O-Alkyl, S-Alkyl, Amino oder substituiertes Amino ist;
R_{E} O oder S ist;
R_{F} H oder eine labile blockierende Gruppe ist;
R_{X} H, OH oder eine Zucker-derivatisierende Gruppe ist;
B_{X} eine natürlich vorkommende oder synthetische Nukleosidbase oder blockierte Nukleosidbase ist;
L eine Abgangsgruppe ist oder L und B_{X} zusammen ein 2-3'- oder 6-3'-Pyrimidin- oder 8-3'-Purin-Cyclo-Nukleosid sind; und
Y eine stabile blockierende Gruppe, ein Festkörperträger, ein Nukleotid auf einem Festkörperträger oder ein Oligonukleotid auf einem Festkörperträger ist.

12. Verfahren nach Anspruch 11, wobei L aus der Gruppe, bestehend aus Halogen, Alkylsulfonyl, substituiertem Alkylsulfonyl, Arylsulfonyl, substituiertem Arylsulfonyl, Heterocyclosulfonyl und Trichloracetimidat, ausgewählt ist.

13. Verfahren nach Anspruch 11, wobei L aus der Gruppe, bestehend aus Chlor, Fluor, Brom, lod, p-(2,4-Dinitroanilin)-benzolsulfonyl, Benzolsulfonyl, Methylsulfonyl (Mesylat), p-Methylbenzolsulfonyl (Tosylat), p-Brombenzolsulfonyl, Trifluormethylsulfonyl (Triflat), Trichloracetimidat, Acyloxy, 2,2,2-Trifluorethansulfonyl, Imidazolsulfonyl und 2,4,6-Trichlorphenyl, ausgewählt ist.

14. Verfahren nach Anspruch 11, wobei die Base aus der Gruppe, bestehend aus Natriumhydrid, Methylmagnesiumchlorid, t-Butylmagnesiumchlorid und Methylmagnesiumfluorid, ausgewählt ist.

15. Verfahren nach Anspruch 11, wobei R_{F} eine säurelabile blockierende Gruppe ist.

16. Verfahren nach Anspruch 11, wobei Y eine hydrogenolyseempfindliche blockierende Gruppe ist.

17. Verfahren nach Anspruch 11, wobei R_{F} aus der Gruppe, bestehend aus t-Butyl, Dimethoxytrityl und Tetrahydropyranyl, ausgewählt ist.

18. Verfahren nach Anspruch 11, wobei das neue erste Synthon die Struktur: 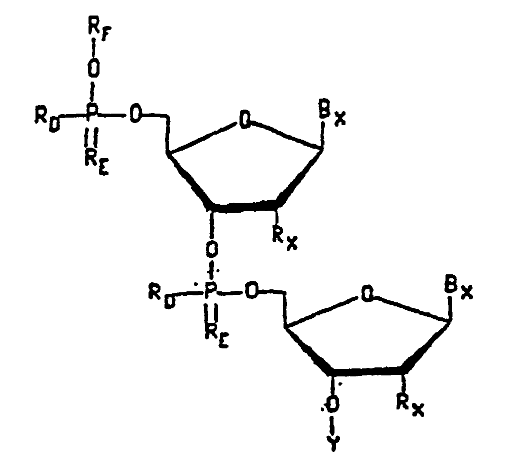 aufweist.

19. Verfahren nach Anspruch 11, ferner umfassend das Inkontaktbringen des ersten Synthons mit der Base in einem Lösungsmittel, das aus der Gruppe, bestehend aus Acetonitril, Tetrahydrofuran und Dioxan, ausgewählt ist.

20. Verfahren nach Anspruch 11, wobei das Reagens, das zum Entfernen der blockierenden Gruppe R_{F} verwendet wird, eine Säure ist, die aus der Gruppe, bestehend aus Trichloressigsäure, Essigsäure und Trifluormethansulfonsäure, ausgewählt ist.

21. Verbindung mit der Struktur: 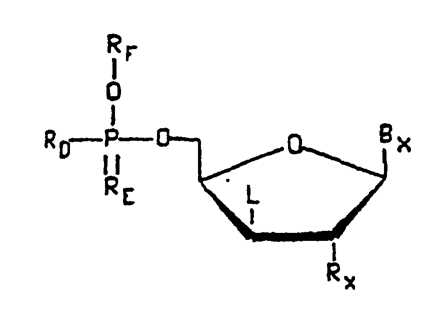 wobei:
Q O oder CH₂ ist;
R_{D} O⁻, S⁻, Methyl, O-Alkyl, S-Alkyl, Amino oder substituiertes Amino ist;
R_{E} O oder S ist;
R_{F} H oder eine blockierende Gruppe ist;
R_{X} H, OH oder eine Zucker-derivatisierende Gruppe ist;
B_{X} eine Nukleosidbase oder eine blockierte Nukleosidbase ist; und
L eine Abgangsgruppe ist oder L und B_{X} zusammen ein 2-3'- oder 6-3'-Pyrimidin- oder 8-3'-Purin-Cyclo-Nukleosid sind.

22. Verbindung nach Anspruch 21, wobei L aus der Gruppe, bestehend aus Halogen, Alkylsulfonyl, substituiertem Alkylsulfonyl, Arylsulfonyl, substituiertem Arylsulfonyl, Heterocyclosulfonyl und Trichloracetimidat, ausgewählt ist.

23. Verbindung nach Anspruch 21, wobei L aus der Gruppe, bestehend aus Chlor, Fluor, Brom, lod, p-(2,4-Dinitroanilin)-benzolsulfonyl, Benzolsulfonyl, Methylsulfonyl (Mesylat), p-Methylbenzolsulfonyl (Tosylat), p-Brombenzolsulfonyl, Trifluormethylsulfonyl (Triflat), Trichloracetimidat, Acyloxy, 2,2,2-Trifluorethansulfonyl, Imidazolsulfonyl und 2,4,6-Trichlorphenyl, ausgewählt ist.

24. Verbindung nach Anspruch 21, wobei R_{X} H, OH, Alkyl, Alkenyl, Alkinyl, substituiertes Alkyl, Alkenyl, Alkinyl, F, Cl, Br, CN, CF₃, OCF₃, OCN, O-Alkyl, O-Alkenyl, O-Alkinyl, substituiertes O-Alkyl, substituiertes O-Alkenyl, substituiertes O-Alkinyl, S-Alkyl, S-Alkenyl, S-Alkinyl, substituiertes S-Alkyl, substituiertes S-Alkenyl, substituiertes S-Alkinyl, SOMe, SO₂Me, ONO₂, NO₂, N₃, NH₂, NH-Alkyl, NH-Alkenyl, NH-Alkinyl, substituiertes NH-Alkyl, substituiertes NH-Alkenyl, substituiertes NH-Alkinyl, OCH₂CH=CH₂, OCH=CH₂, OCH₂CCH, OCCH, Aralkyl, Aralkenyl, Aralkinyl, Heteroaralkyl, Heteroaralkenyl, Heteroaralkinyl, Heterocycloalkyl, Poly(alkylamino), substituiertes Silyl, ein RNAspaltender Rest, eine Gruppe, welche die pharmakodynamischen Eigenschaften eines Oligonukleotids verbessert, oder eine Gruppe, welche die pharmakokinetischen Eigenschaften eines Oligonukleotids verbessert, ist.

25. Verbindung nach Anspruch 21, wobei R_{F} aus der Gruppe, bestehend aus H, t-Butyl, Dimethoxytrityl und Tetrahydropyranyl, ausgewählt ist.

26. Verbindung nach Anspruch 21, wobei Q O ist.

27. Verbindung nach Anspruch 21 mit Sp-Stereochemie.

28. Verbindung nach Anspruch 21 mit Rp-Stereochemie.

29. Enzymatisches Verfahren zum Synthetisieren des Oligonukleotids nach Anspruch 2, wobei das Oligonukleotid mindestes 10 Nukleosid-Einheiten aufweist, die durch entweder im wesentlichen ausschließlich Sp- oder im wesentlichen ausschließlich Rp-Phosphor-Interzucker-Verknüpfungen miteinander verbunden sind, umfassend die Schritte:
Kombinieren eines Sequenzprimers und eines Templates;
Hinzugeben eines Überschusses aller vier Nukleosid-5'-O-Triphosphate, die einen gewünschten 5'-Phosphat-Rest aufweisen; wobei das Triphosphat eines der im wesentlichen ausschließlich Rp- oder ausschließlich Sp-Triphosphate ist;
Hinzugeben von Polymerase, die wirksam für das Verlängern des Primers ist, um ein Oligonukleotid zu bilden, das komplementär zu dem Templat ist; und
Disassoziieren des entstehenden Oligonukleotids von dem Primer und von dem Templat.

30. Verfahren nach Anspruch 29, wobei mindestens ein Schritt eine Vielzahl von Malen wiederholt wird, die ausreicht, um die Ausbeute des sequenzspezifischen Phosphorthioats zu fördern.

31. Enzymatisches Verfahren zum Synthetisieren des Oligonukleotids nach Anspruch 2, wobei das Oligonukleotid mindestes 10 Nukleosid-Einheiten aufweist, die durch entweder im wesentlichen ausschließlich Sp-Phosphor-Interzucker-Verknüpfungen oder im wesentlichen ausschließlich Rp-Phosphor-Interzucker-Verknüpfungen miteinander verbunden sind, umfassend die Schritte:
Vereinigen eines Sequenzprimers und eines Templates;
Hinzugeben eines Überschusses einer racemischen Mischung aller vier Nukleosid-5'-O-(Triphosphate), die einen gewünschten 5'-Phosphat-Rest aufweisen;
Hinzugeben eines ausgewählten Metallions, das wirksam ist, die bevorzugte Einfügung von entweder Rp- oder Sp-Triphosphaten zu fördern;
Hinzugeben von Polymerase, die wirksam für das Verlängern des Primers ist, um ein Oligonukleotid zu bilden, das komplementär zu dem Templat ist; und
Disassoziieren des entstehenden Oligonukleotids von dem Primer und von dem Templat.

32. Verfahren nach Anspruch 31, wobei das Metallion Magnesium ist.

33. Verfahren nach Anspruch 31, wobei das Metallion Mangan ist.

34. Verfahren nach Anspruch 29 oder Anspruch 31, ferner umfassend die Schritte:
Prähybridisieren des Templats und des Primers durch Erwärmen des Templats und des Primers miteinander;
Abkühlen der Templat-und-Primer-Mischung vor der Zugabe der Triphosphate.

35. Verfahren nach Anspruch 29 oder Anspruch 31, wobei die Polymerase T7-DNA-Polymerase, modifizierte T7-DNA-Polymerase I, T7-RNA-Polymerase, T4-Bakteriophagen-Polymerase, modifizierte T4-DNA-Polymerase, *M. luteus*-Polymerase, DNA-poly-Klenow-Fragment-Polymerase, *E. coli*-RNA-Polymerase oder *E. coli*-DNA-Polymerase ist.

36. Verfahren nach Anspruch 29 oder Anspruch 31, wobei die Disassozierung von dem Primer mittels Pst-1-Restriktions-Endonuklease, BamH1-Restriktions-Endonuklease, Smal-Restriktions-Endonuklease oder HinD-III-Restriktions-Endonuklease erreicht wird.

37. Verfahren nach Anspruch 29 oder Anspruch 31, wobei die Disassozierung durch DNAse-I-Verdauung erreicht wird.

## Revendications

1. Oligonucléotide spécifique de séquence, comportant au moins 10 unités liées ensemble et complémentaire d'au moins une partie d'une séquence d'un ARN ou ADN ciblé, dans lequel au moins trois desdites unités nucléosidiques sont liées ensemble par un phosphorothioate, alkylphosphonate, phosphotriester ou phosphoramidate soit pratiquement tout *R*ₚ, soit pratiquement tout *S*ₚ, étant entendu que si ledit oligonudéotide est assemblé par des chaînons de liaisons phosphorothioate, ledit oligonucléotide comporte moins de la totalité desdites unités nucléosidiques liées ensemble par des liaisons soit tout *R*ₚ, soit tout *S*ₚ.

2. Oligonucléotide de la revendication 1, dans lequel lesdits chaînons de liaison phosphate sont choisis dans le groupe constitué par un phosphorothioate *S*ₚ chiral, un phosphorothioate *R*ₚ chiral, un alkylphosphonate *S*ₚ chiral, un alkylphosphonate *R*ₚ chiral, un phosphoramidate *S*ₚ chiral, un phosphoramidate *R*ₚ chiral, un phosphotriester *S*ₚ chiral et un phosphotriester *R*ₚ chiral.

3. Oligonucléotide comprenant au moins 10 unités nucléosldiques liées ensemble par des chaînons de liaison phosphate, dans lequel :
au moins l'une des unités nudéosidiques est une unité nucléosidique non rencontrée dans la nature, comportant en position 2' un substituant choisi dans l'ensemble constitué par les groupes OH, alkyle en C₁₋₁₂, alkyle en C₁₋₁₂ substitué, F, Cl, Br, CN, CF₃, OCF₃, OCN, O-alkyle, O-alkyle substitué, S-alkyle, S-alkyle substltué, SOMe, SO₂Me, ONO₂, NO₂, N₃, NH₂, NH-alkyle, NH-alkyle substitué, OCH₂CH=CH₂, OCH=CH₂, OCH₂CCH, OCCH, aralkyle, hétéroalkyle, hétérocycloalkyle, polyalkylamino, silyle substitué, une entité coupant l'ARN, un groupe améliorant les propriétés pharmacodynamiques d'un oligonucléotide, et un groupe améliorant les propriétés pharmacocinétiques d'un oligonucléotide ; et
au moins deux des unités sont reliées par des chaînons de liaison phosphate chiraux.

4. Oligonudéotide de la revendication 3, dans lequel lesdits chaînons de liaison phosphate chiraux sont choisis dans le groupe constitué par un phosphorothioate *S*ₚ chiral, un phosphorothioate *R*ₚ chiral, un alkylphosphonate *S*ₚ chiral, un alkylphosphonate *R*ₚ chiral, un phosphoramidate *S*ₚ chiral, un phosphoramidate *R*ₚ chiral, un phosphotriester *S*ₚ chiral et un phosphotriester *R*ₚ chiral.

5. Oligonucléotide de la revendication 3, dans lequel chacun des chaînons de liaison phosphate est un chaînon de liaison phosphate chiral.

6. Oligonucléotide selon l'une quelconque des revendications 1 à 5, comprenant une pluralité d'unités nucléosidiques reliées entre elles par des chaînons de liaison phosphotriester tout *S*ₚ ou par des chaînons de liaison phosphotriester tout *R*ₚ.

7. Oligonucléotide selon l'une quelconque des revendications 1 à 5, comprenant une pluralité d'unités nucléosidiques reliées entre elles par des chaînons de liaison phosphoramidate tout *S*ₚ ou par des chaînons de liaison phosphoramldate tout *R*ₚ.

8. Oligonucléotide selon l'une quelconque des revendications 1 à 5, comprenant au moins 10 unités nucléosidiques reliées entre elles par des chaînons de liaison alkylphosphonate tout *S*ₚ ou par des chaînons de liaison alkylphosphonate tout *R*ₚ.

9. Oligonudéotide de la revendication 8, dans lequel lesdits chaînons de liaisons alkylphosphonate sont des chaînons de liaison méthylphosphonate.

10. Oligonucléotide selon l'une quelconque des revendications 1 à 9, pour utilisation en médecine.

11. Procédé pour la synthèse des oligonucléotides de la revendication 1, comprenant les étapes suivantes :
(a) sélection d'un premier composant de synthèse de formule: 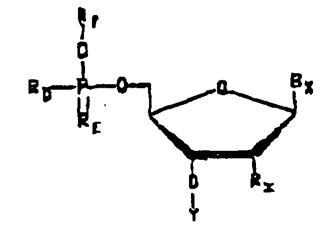
(a) sélection d'un second composant de synthèse de formule: 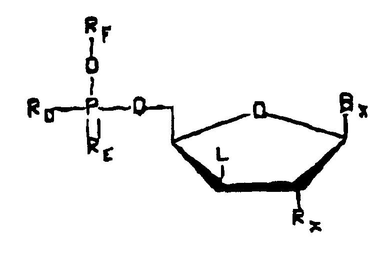
(c) mise en contact du second composant de synthèse avec le premier composant de synthèse en présence d'une base, pour effectuer l'attaque nucléophile d'un 5'-phosphate du premier composant de synthèse en une position 3' du second composant de synthèse, pour l'obtention d'un nouveau premier composant de synthèse par une inversion de la configuration en la position 3' du second composant de synthèse ; et
(d) traitement du nouveau premier composant de synthèse par un réactif pour éliminer le groupe bloquant R_{F};
où :
Q est O ou CH₂ ;
R_{D} est un groupe méthyle, O-alkyle, S-alkyle, amino ou amino substitué ;
R_{E} est O ou S ;
R_{F} est H ou un groupe bloquant labile;
Rₓ est H, OH ou un groupe transformant un sucre en dérivé ;
Bₓ est une base nudéosidique rencontrée dans la nature ou synthétique, ou une base nudéosidique bloquée;
L est un groupe partant, ou L et Bₓ forment ensemble un 2-3'- ou 6-3'-cyclonucléoside pyrimidique ou un 8-3'-cyclonucléoside purique; et
Y est un groupe bloquant stable, un support à l'état solide, un nudéotide sur un support à l'état solide, ou un oligonucléotide sur un support à l'état solide.

12. Procédé de la revendication 11, dans lequel L est choisi dans l'ensemble constitué par un atome d'halogène et un groupe alkylsulfonyle, alkylsulfonyle substitué, arylsulfonyle, arylsulfonyle substitué, hétérocyclosulfonyle ou trichloroacétimidate.

13. Procédé de la revendication 11, dans lequel L est choisi dans l'ensemble constitué par les atomes de chlore, fluor, brome, iode et les groupes p-(2,4-dinitroanilino)benzenesulfonyle, benzènesulfonyle, méthylsulfonyle (mésylate), p-méthylbenzènesulfonyle (tosylate), p-bromobenzènesulfonyle, trifluorométhylsulfonyle (triflate), trichloracétimidate, acyloxy, 2,2,2-trifluoroéthanesulfonyle, imidazolesulfonyle et 2,4,6-trichlorophényle.

14. Procédé de la revendication 11, dans lequel ladite base est choisie dans l'ensemble constitué par l'hydrure de sodium, le chlorure de méthylmagnésium, le chlorure de tert-butylmagnésium et le fluorure de méthylmagnésium.

15. Procédé de la revendication 11, dans lequel R_{F} est un groupe bloquant labile en présence d'acides.

16. Procédé de la revendication 11, dans lequel Y est un groupe bloquant sensible à l'hydrogénolyse.

17. Procédé de la revendication 11, dans lequel R_{F} est choisi dans l'ensemble constitué par les groupes tert-butyle, diméthoxytrityle et tétrahydropyrannyle.

18. Procédé de la revendication 11, dans lequel le nouveau premier composant de synthèse correspond à la formule:

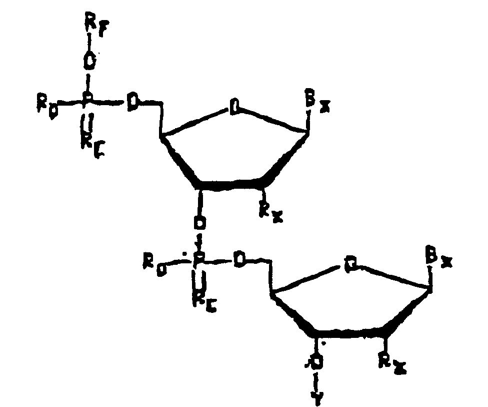

19. Procédé de la revendication 11, comprenant en outre la mise en contact dudit premier composant de synthèse avec ladite base dans un solvant choisi dans l'ensemble constitué par l'acétonitrile, le tétrahydrofuranne et le dioxanne.

20. Procédé de la revendication 11, dans lequel ledit réactif utilisé pour éliminer le groupe bloquant R_{F} est un acide choisi dans l'ensemble constitué par les acides trichloracétique, acétique et trifluorométhanesulfonlque.

21. Composé de formule : 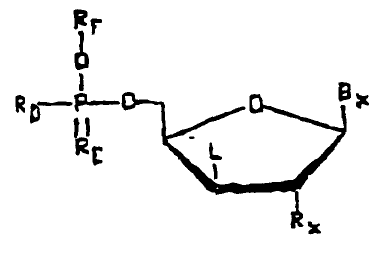 dans laquelle :
Q est O ou CH₂ ;
R_{D} est O^{⊖}, S^{⊖}, un groupe méthyle, O-alkyle, S-alkyle, amino ou amino substitué;
R_{E} est O ou S ;
R_{F} est H ou un groupe bloquant ;
Rₓ est H, OH ou un groupe transformant un sucre en dérivé ;
Bₓ est une base nudéosidique ou une base nucléosidique bloquée;
L est un groupe partant, ou L et Bₓ forment ensemble un 2-3'- ou 6-3'-cyclonucléoside pyrimidique ou un 8-3'-cyclonucléoside purique ;

22. Composé de la revendication 21, dans lequel L est choisi dans l'ensemble constitué par un atome d'halogène et un groupe alkylsulfonyle, alkylsulfonyle substitué, arylsulfonyle, arylsulfonyle substitué, hétérocyclosulfonyle ou trichloroacétimidate.

23. Procédé de la revendication 21, dans lequel L est choisi dans l'ensemble constitué par les atomes de chlore, fluor, brome, iode et les groupes p-(2,4-dinitroanllino)benzènesulfonyle, benzènesulfonyle, méthylsulfonyle (mésylate), p-méthylbenzènesulfonyle (tosylate), p-bromobenzènesulfonyle, trifluorométhylsulfonyle (triflate), trichloracétimidate, acyloxy, 2,2,2-trifluoroéthanesulfonyle, imidazolesulfonyle et 2,4,6-trichlorophényle.

24. Composé de la revendication 21, dans lequel Rₓ est H, OH, un groupe alkyle, alcényle, alcynyle, un groupe alkyle, alcényle, alcynyle substitué, F, Cl, Br, CN, CF₃, OCF₃, OCN, O-alkyle, O-alcényle, O-alcynyle, O-alkyle substitué, O-alcényle substitué, O-alcynyle substitué, S-alkyle, S-alcényle, S-alcynyle, S-alkyle substitué, S-alcényle substitué, S-alcynyle substitué, SOMe, SO₂Me, ONO₂, NO₂, N₃, NH₂, NH-alkyle, NH-alcényle, NH-alcynyle, NH-alkyle substitué, NH-alcényle substitué, NH-alcynyle substitué, OCH₂CH=CH₂, OCH=CH₂, OCH₂CCH, OCCH, aralkyle, aralcényle, aralcynyle, hétéroaralkyle, hétémalcényle, hétéroaralcynyle, hétérocycloalkyle, poly(alkylamino), silyle substitué, une entité coupant l'ARN, un groupe destiné à améliorer les propriétés pharmacodynamiques d'un oligonucléotide ou un groupe destiné à améliorer les propriétés pharmacocinétiques d'un oligonucléotide.

25. Composé de la revendication 21, dans lequel R₇ est choisi dans l'ensemble constitué par H et les groupes tert-butyle, diméthoxytrityle et tétrahydropyrannyle.

26. Composé de la revendication 21, dans lequel R₇ est choisi Q est O.

27. Composé de la revendication 21, ayant une configuration stéréochimique *S*ₚ.

28. Composé de la revendication 21, ayant une configuration stéréochimique *R*ₚ.

29. Procédé enzymatique pour la synthèse de l'oligonudéotide de la revendication 2, ledit oligonudéotide comportant au moins 10 unités nucléosidlques reliées entre elles par des chaînons de liaison Inter-sucres phosphorés soit pratiquement tout *S*ₚ, soit pratiquement tout *R*ₚ, comprenant les étapes suivantes :
réunion d'une amorce de séquence et d'une matrice;
addition d'un excès de tous les quatre nucléoside-5'-o-triphosphates comportant un fragment 5'-phosphate désiré ; ledit triphosphate étant l'un des triphosphates pratiquement tout *R*ₚ ou tout *S*ₚ ;
addition d'une polymérase efficace pour l'extension de l'amorce pour former un oligonucléotide complémentaire de ladite matrice ; et
séparation de l'oligonucléotide résultant d'avec ladite amorce et d'avec la matrice.

30. Procédé de la revendication 29, dans lequel au moins une étape est répétée un nombre de fois suffisant pour augmenter le rendement en ledit phosphorothioate-oligonudéotide spécifique de séquence.

31. Procédé enzymatique pour la synthèse de l'oligonudéotide de la revendication 2, ledit oligonucléotide comportant au moins 10 unités nucléosidiques reliées entre elles par soit des chaînons de liaison inter-sucres phosphorés pratiquement tout *S*ₚ, soit des chaînons de liaison inter-sucres phosphorés pratiquement tout *R*ₚ, comprenant les étapes suivantes :
réunion d'une amorce de séquence et d'une matrice ;
addition d'un excès d'un mélange racémique de tous les quatre nucléoside-5'-o-(triphosphates) comportant un fragment 5'-phosphate désiré ;
addition d'un ion métallique choisi, efficace pour favoriser l'incorporation préférentielle de triphosphates soit *R*ₚ, soit *S*ₚ;
addition d'une polymérase efficace pour l'extension de l'amorce pour former un oligonucléotide complémentaire de ladite matrice ; et
séparation dudit oligonucléotide résultant d'avec ladite amorce et d'avec la matrice.

32. Procédé de la revendication 31, dans lequel ledit ion métallique est l'ion magnésium.

33. Procédé de la revendication 31, dans lequel ledit ion métallique est l'ion manganèse.

34. Procédé de la revendication 29 ou de la revendication 31, comprenant en outre les étapes suivantes :
préhybridation de ladite matice avec ladite amorce par chauffage de ladite matrice et de ladite amorce ensemble; et
refroidissement dudit mélange de matrice et d'amorce avant l'addition desdits triphosphates.

35. Procédé de la revendication 21 ou de la revendication 31 dans lequel ladite polymérase est l'ADN polymérase de T7, une ADN polymérase de T7 modifiée, l'ARN polymérase de T7, la polymérase de bactériophage T4, une ADN polymérase de T4 modifiée, la polymérase de *M*. *luteus*, le fragment de Klenow d'ADN polymérase, l'ARN polymérase de *E*. *coli* ou l'ADN polymérase de *E*. *coli.*

36. Procédé de la revendication 29 ou de la revendication 31, dans lequel la séparation d'avec l'amorce est effectuée au moyen de l'endonucléase de restriction *Pst*I, de l'endonucléase de restriction *Sma*I ou de l'endonudéase de restriction *Hin*dIII.

37. Procédé de la revendication 29 ou de la revendication 31, dans lequel la séparation est effectuée par digestion par la DNase I.
